# EUROPEAN PATENT APPLICATION

(11) **EP 2 022 854 A1**
(43) Date of publication of application: **11.02.2009**
(21) Application number: 07743581.6
(22) Date of filing: 17.05.2007
(51) Int. Cl.: C12N 15/09, C07K 16/18, C12N 15/02, C12P 21/08

(54) **PHAGE-DISPLAYING SINGLE-CHAIN ANTIBODY CAPABLE OF RECOGNIZING NON-REDUCED MANNOSE RESIDUE**

(30) Priority: 17.05.2006 JP 2006137604
(71) Applicant: TOKAI UNIVERSITY EDUCATIONAL SYSTEM, Shibuya-ku Tokyo 1510063 (JP); Keio University, Tokyo 108-8345 (JP)
(72) Inventor: YAMAGUCHI, Yoko, Hiratsuka-shi Kanagawa 259-1207 (JP); NAKATA, Munehiro, Hiratsuka-shi Kanagawa 259-1207 (JP); SAKAI, Keiko, Hiratsuka-shi Kanagawa 259-1207 (JP); SHIMIZU, Yoshitaka, Hiratsuka-shi Kanagawa 259-1207 (JP); TAKASAKI, Ayano, Hiratsuka-shi Kanagawa 259-1207 (JP); CHIBA, Tomoki, Hiratsuka-shi Kanagawa 259-1207 (JP); KUSADA, Yu, Hiratsuka-shi Kanagawa 259-1207 (JP); SHIMIZU, Nobuyoshi, Tokyo 160-8582 (JP); TAKAYANAGI, Atsushi, Tokao 160-8582 (JP)
(74) Representative: Polypatent
(86) International application number: PCT/JP2007/060146
(87) International publication number: WO 2007/132917

(57) **Abstract**

It is an object of the present invention to provide antibodies which recognize non-reducing mannose residues. The present invention provides antibodies recognizing non-reducing mannose residues, which are obtained through screening of phage-display library of human scFvs by using, as an antigen, Man3-DPPE which is an artificial glycolipid synthesized from mannotriose and dipalmitoylphosphatidylethanolamine by reductive amination.

SEQUENCE LISTING

<110>  Tokai University Educational System
       Keio University

<120>  Phage-displaying single-chain antibody capable of recognizing
       non-reduced mannose residue

<130>  P22295EP

<140>  EP 07 743 581.6
<141>  2007-05-17

<150>  JP20060137604
<151>  2006-05-17

<160>  110

<170>  PatentIn version 3.3

<210>  1
<211>  354
<212>  DNA
<213>  Homo sapiens

<400>  1
caggtgacct tgaaggagtc tggtcctgcg ctggtgaaac ccacacagac cctcacactg      60

acctgcacct tctctgactt ctcactcacc actagtggaa tgcgtgtcag ctggatccgt     120

cagcccccag ggaaggccct ggagtggctt gcacgcattg attgggatga tgataagttc     180

tacagcacct ctctgaagac caggctcacc atctccaagg acacctccaa aaaccaggtg     240

gtccttacac tgaccaacat ggaccctgtg gacacagcca cgtattactg tgcacgggat     300

atttcgaaag cacatgacta ctggggccag ggaaccctgg tcactgtctc ctca           354


<210>  2
<211>  118
<212>  PRT
<213>  Homo sapiens

<400>  2

Gln Val Thr Leu Lys Glu Ser Gly Pro Ala Leu Val Lys Pro Thr Gln
1               5                   10                  15


Thr Leu Thr Leu Thr Cys Thr Phe Ser Asp Phe Ser Leu Thr Thr Ser
            20                  25                  30


Gly Met Arg Val Ser Trp Ile Arg Gln Pro Pro Gly Lys Ala Leu Glu
        35                  40                  45


Trp Leu Ala Arg Ile Asp Trp Asp Asp Asp Lys Phe Tyr Ser Thr Ser
    50                  55                  60


Leu Lys Thr Arg Leu Thr Ile Ser Lys Asp Thr Ser Lys Asn Gln Val
65                  70                  75                  80


Val Leu Thr Leu Thr Asn Met Asp Pro Val Asp Thr Ala Thr Tyr Tyr
                85                  90                  95


Cys Ala Arg Asp Ile Ser Lys Ala His Asp Tyr Trp Gly Gln Gly Thr
            100                 105                 110


Leu Val Thr Val Ser Ser
        115


<210>  3

<211>  324
<212>  DNA
<213>  Homo sapiens

<400>  3
gaaacgacac tcacgcagtc tccagccacc ctgtctgtgt ctctagggga cagagcctcc      60

ctctcctgca gggccagtca gagtgtgggc accaagttag cctggtacca gcacaaacct     120

ggccaggctc ccaggctcct cttctatggt gcatccacca gggcccctag tgtcccggcc     180

aggttcagtg gcagtggctc tgggacagaa ttcactctca ccatcagcag cctgcagtct     240

gaagattttg cagtttatta ctgtcagcaa tatataaact ggcctcggac gttcggccaa     300

gggaccaagg tggaaatcaa aggc                                            324


<210>  4
<211>  108
<212>  PRT
<213>  Homo sapiens

<400>  4

Glu Thr Thr Leu Thr Gln Ser Pro Ala Thr Leu Ser Val Ser Leu Gly
1               5                   10                  15

Asp Arg Ala Ser Leu Ser Cys Arg Ala Ser Gln Ser Val Gly Thr Lys
            20                  25                  30

Leu Ala Trp Tyr Gln His Lys Pro Gly Gln Ala Pro Arg Leu Leu Phe
        35                  40                  45

Tyr Gly Ala Ser Thr Arg Ala Pro Ser Val Pro Ala Arg Phe Ser Gly
    50                  55                  60

Ser Gly Ser Gly Thr Glu Phe Thr Leu Thr Ile Ser Ser Leu Gln Ser
65                  70                  75                  80

Glu Asp Phe Ala Val Tyr Tyr Cys Gln Gln Tyr Ile Asn Trp Pro Arg
                85                  90                  95

Thr Phe Gly Gln Gly Thr Lys Val Glu Ile Lys Gly
            100                 105


<210>  5
<211>  390
<212>  DNA
<213>  Homo sapiens

<400>  5
caggtgcagc tacagcagtg gggcgcggga atattgaagc cttcggagac cctgtctcta      60

acctgcgcta tctacggcac atatatcggt ggttatcact ggacgtggat ccgacggccc     120

ccaggggggg gactggagtg gattggagaa atcgaaaata ctggtaacat caaatataat     180

ccgtccctca ggaatcgagt caccatttcc tcagacacgt ccaaaagaca agtcttcttg     240

agtctgactt ctctgaccac tgcggacacg gctgtatatt attgtgcgaa gggggggggt     300

aaagaggtca cggttctcgg cctgcctgtg gggtgggact actacggcat ggacatatgg     360

ggcccaggga ccacggtcac cgtctcctca                                      390


<210>  6
<211>  130
<212>  PRT
<213>  Homo sapiens

<400> 6

Gln Val Gln Leu Gln Gln Trp Gly Ala Gly Ile Leu Lys Pro Ser Glu
1               5                   10                  15

Thr Leu Ser Leu Thr Cys Ala Ile Tyr Gly Thr Tyr Ile Gly Gly Tyr
            20                  25                  30

His Trp Thr Trp Ile Arg Arg Pro Pro Gly Gly Gly Leu Glu Trp Ile
        35                  40                  45

Gly Glu Ile Glu Asn Thr Gly Asn Ile Lys Tyr Asn Pro Ser Leu Arg
    50                  55                  60

Asn Arg Val Thr Ile Ser Ser Asp Thr Ser Lys Arg Gln Val Phe Leu
65                  70                  75                  80

Ser Leu Thr Ser Leu Thr Thr Ala Asp Thr Ala Val Tyr Tyr Cys Ala
                85                  90                  95

Lys Gly Gly Gly Lys Glu Val Thr Val Leu Gly Leu Pro Val Gly Trp
            100                 105                 110

Asp Tyr Tyr Gly Met Asp Ile Trp Gly Pro Gly Thr Thr Val Thr Val
        115                 120                 125

Ser Ser
    130

<210> 7
<211> 324
<212> DNA
<213> Homo sapiens

<400> 7
gacatccaga tgacccagtc tccatcctcc ctgtctgcat ctgtgggaga cagagtcacc      60

atcacttgcc gggcaagtca gacccttacc acctatttaa attggtatca acagagacca     120

gggaaagccc caaaactcct catctatctt gcatccactt tggacagtgg ggtcccatca     180

agattcagtg gcagtgcatc tggcacaact ttcaccctca ccatcaccag tctacaacct     240

gacgatgttg caacttatta ctgtcaacag agttacaata ttcctcggac gttcggccag     300

gggaccaagg tggagatcaa aggc                                            324

<210> 8
<211> 108
<212> PRT
<213> Homo sapiens

<400> 8

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Thr Leu Thr Thr Tyr
            20                  25                  30

Leu Asn Trp Tyr Gln Gln Arg Pro Gly Lys Ala Pro Lys Leu Leu Ile
        35                  40                  45

Tyr Leu Ala Ser Thr Leu Asp Ser Gly Val Pro Ser Arg Phe Ser Gly
    50                  55                  60

Ser Ala Ser Gly Thr Thr Phe Thr Leu Thr Ile Thr Ser Leu Gln Pro
65                  70                  75                  80

Asp Asp Val Ala Thr Tyr Tyr Cys Gln Gln Ser Tyr Asn Ile Pro Arg
                85                  90                  95

Thr Phe Gly Gln Gly Thr Lys Val Glu Ile Lys Gly
            100                 105

<210>  9
<211>  354
<212>  DNA
<213>  Homo sapiens

<400>  9
caggtgacct tgaaggagtc tggtcctacg ctggtgaaac ccacacagac cctcacgctg      60

acctgcacct tctctggatt ctcactcagc actagtggaa tgggtgtggg ctggatccgt     120

cagcccccag gaaaggccct ggagtggctt gcagtcattt attgggatga tgataagcgc     180

tacagcccat ctctgaagac caggctcacc atcaccaagg acacctccaa aaaccaggtt     240

gtccttacaa tgaccaacat ggaccctgtg gacacagcca catattactg tgcacagcaa     300

cggtggtcca actttgacta ctggggccag ggaatcctgg tcaccgtctc ctca           354

<210>  10
<211>  118
<212>  PRT
<213>  Homo sapiens

<400>  10

Gln Val Thr Leu Lys Glu Ser Gly Pro Thr Leu Val Lys Pro Thr Gln
1               5                   10                  15

Thr Leu Thr Leu Thr Cys Thr Phe Ser Gly Phe Ser Leu Ser Thr Ser
            20                  25                  30

Gly Met Gly Val Gly Trp Ile Arg Gln Pro Pro Gly Lys Ala Leu Glu
        35                  40                  45

Trp Leu Ala Val Ile Tyr Trp Asp Asp Asp Lys Arg Tyr Ser Pro Ser
    50                  55                  60

Leu Lys Thr Arg Leu Thr Ile Thr Lys Asp Thr Ser Lys Asn Gln Val
65                  70                  75                  80

Val Leu Thr Met Thr Asn Met Asp Pro Val Asp Thr Ala Thr Tyr Tyr
                85                  90                  95

Cys Ala Gln Gln Arg Trp Ser Asn Phe Asp Tyr Trp Gly Gln Gly Ile
            100                 105                 110

Leu Val Thr Val Ser Ser
        115

<210>  11
<211>  327
<212>  DNA
<213>  Homo sapiens

<400>  11
gaaattgtat tgacgcagtc tccagccacc ctgtctttgt ctccagggga aagagccacc      60

ctctcctgca gggccagtca gagtgttagc ggcaacttag cctggtacca gcagaaacct 120

ggccaggctc ccaggctcct catctttgct acatccacca gggccactgg tatcccagtc 180

aggtttagtg gccgtggctc tgggacagac ttcactctca ccatcagcag actggagcct 240

gaagattttg cagtgtatta ctgtcagcag tatggtggct cacctcggct cactttcggc 300

ggagggacca aggtggaaat caaaggc 327

<210> 12
<211> 109
<212> PRT
<213> Homo sapiens

<400> 12

Glu Ile Val Leu Thr Gln Ser Pro Ala Thr Leu Ser Leu Ser Pro Gly
1               5                   10                  15

Glu Arg Ala Thr Leu Ser Cys Arg Ala Ser Gln Ser Val Ser Gly Asn
            20                  25                  30

Leu Ala Trp Tyr Gln Gln Lys Pro Gly Gln Ala Pro Arg Leu Leu Ile
        35                  40                  45

Phe Ala Thr Ser Thr Arg Ala Thr Gly Ile Pro Val Arg Phe Ser Gly
    50                  55                  60

Arg Gly Ser Gly Thr Asp Phe Thr Leu Thr Ile Ser Arg Leu Glu Pro
65                  70                  75                  80

Glu Asp Phe Ala Val Tyr Tyr Cys Gln Gln Tyr Gly Gly Ser Pro Arg
                85                  90                  95

Leu Thr Phe Gly Gly Gly Thr Lys Val Glu Ile Lys Gly
            100                 105

<210> 13
<211> 366
<212> DNA
<213> Homo sapiens

<400> 13
caggtgcagc tacagcagtg gggcgcagga ctgttgaagc cttcggagac cctgtccctc 60

acctgcgctg tttctggtta tgggtccctc agtggttact actggagctg gatccgccag 120

cccccaggga aggggctgga gtggattggg gaaatcaatc gtggcggaag caccaactac 180

aacccgtccc tcaagagtcg aatcaccata tcagtagaca cgtccaagaa ccagttctcc 240

ctgaatctga actctgtcac cgccgcggac acggctgtgt attactgtgc gagaagtggc 300

ctgtatggca ccactccgtc aacctttgac cactggggcc agggaaccct ggtcaccgtc 360

tcctca 366

<210> 14
<211> 122
<212> PRT
<213> Homo sapiens

<400> 14

Gln Val Gln Leu Gln Gln Trp Gly Ala Gly Leu Leu Lys Pro Ser Glu
1               5                   10                  15

Thr Leu Ser Leu Thr Cys Ala Val Ser Gly Tyr Gly Ser Leu Ser Gly

20                  25                  30

Tyr Tyr Trp Ser Trp Ile Arg Gln Pro Pro Gly Lys Gly Leu Glu Trp
        35                  40                  45

Ile Gly Glu Ile Asn Arg Gly Gly Ser Thr Lys Tyr Asn Pro Ser Leu
    50                  55                  60

Lys Ser Arg Ile Thr Ile Ser Val Asp Thr Ser Lys Asn Gln Phe Ser
65                  70                  75                  80

Leu Asn Leu Asn Ser Val Thr Ala Ala Asp Thr Ala Val Tyr Tyr Cys
                85                  90                  95

Ala Arg Ser Gly Leu Tyr Gly Thr Thr Pro Ser Thr Phe Asp His Trp
            100                 105                 110

Gly Gln Gly Thr Leu Val Thr Val Ser Ser
        115                 120

<210>  15
<211>  336
<212>  DNA
<213>  Homo sapiens

<400>  15
cagtctgtgg tgacgcagcc gccctcagtg tctgggaccc caggatcgtg ggtcaccatc      60

tcctgcactg ggaccagctc caacatcggg gcacctaagg gtgtccactg gtaccaacac     120

gtcccaggga cagcccctaa actcctcatc tatgatagta atagtcggtc ctcgggggtc     180

cctgcccgct tctctggctc caagtctggc acttcaggca gcctggccat cgctggactg     240

caacctgagg atgaggctga ttattattgc cagtctcttg ataccaactt gaatgcttgg     300

gtgttcggcg gggggaccaa gctgaccgtc ctaggc                               336

<210>  16
<211>  112
<212>  PRT
<213>  Homo sapiens

<400>  16

Gln Ser Val Val Thr Gln Pro Pro Ser Val Ser Gly Thr Pro Gly Ser
1               5                   10                  15

Trp Val Thr Ile Ser Cys Thr Gly Thr Arg Ser Asn Ile Gly Ala Pro
            20                  25                  30

Lys Gly Val His Trp Tyr Gln His Val Pro Gly Thr Ala Pro Lys Leu
        35                  40                  45

Leu Ile Tyr Asp Ser Asn Ser Arg Ser Ser Gly Val Pro Ala Arg Phe
    50                  55                  60

Ser Gly Ser Lys Ser Gly Thr Ser Gly Ser Leu Ala Ile Ala Gly Leu
65                  70                  75                  80

Gln Pro Glu Asp Glu Ala Asp Tyr Tyr Cys Gln Ser Leu Asp Thr Asn
                85                  90                  95

Leu Asn Ala Trp Val Phe Gly Gly Gly Thr Lys Leu Thr Val Leu Gly
            100                 105                 110

<210> 17
<211> 366
<212> DNA
<213> Homo sapiens

<400> 17
caggtgcagc tgcagcagtc gggcccagga ctggtgaagg cttcggagac cctgtccctc 60

acctgcgctg tctctggtta ctccatcagc agcggttact actggggctg gatccggcag 120

cccccaggga agggactgga gtggattgga aatatctatc atagtgggaa cacctactac 180

aacccgtccc tcaagagtcg agttaccatt tcacgagaca cgtccaagaa tcacttctcc 240

ctgaagctga agtctgtgac cgccgcagac acggccgtgt attactgtgc gagagccgac 300

cccggctata tgatctttaa ctggttcgac ccctggggcc agggaaccct ggtcaccgtc 360

tcctca 366

<210> 18
<211> 122
<212> PRT
<213> Homo sapiens

<400> 18

Gln Val Gln Leu Gln Gln Ser Gly Pro Gly Leu Val Lys Ala Ser Glu
1               5                   10                  15

Thr Leu Ser Leu Thr Cys Ala Val Ser Gly Tyr Ser Ile Ser Ser Gly
            20                  25                  30

Tyr Tyr Trp Gly Trp Ile Arg Gln Pro Pro Gly Lys Gly Leu Glu Trp
        35                  40                  45

Ile Gly Asn Ile Tyr His Ser Gly Asn Thr Tyr Tyr Asn Pro Ser Leu
    50                  55                  60

Lys Ser Arg Val Thr Ile Ser Arg Asp Thr Ser Lys Asn His Phe Ser
65                  70                  75                  80

Leu Lys Leu Lys Ser Val Thr Ala Ala Asp Thr Ala Val Tyr Tyr Cys
                85                  90                  95

Ala Arg Ala Asp Pro Gly Tyr Met Ile Phe Asn Trp Phe Asp Pro Trp
            100                 105                 110

Gly Gln Gly Thr Leu Val Thr Val Ser Ser
        115                 120

<210> 19
<211> 324
<212> DNA
<213> Homo sapiens

<400> 19
aattttatgc tgactcagcc accctcagcg tctgggaccc ccgggcagag ggtcaccatc 60

tcttgttctg gaagcagctc caacatcgga aataatgatg tatactggta ccagcaattc 120

ccaggaacgg cccccaaact cctcatctat aggaataatc agcggccctc aggggtccct 180

gaccgattct ctggctccaa gtctggcacc tcagcctccc tggccatcag tgggctccgg 240

tccgaggatg aggctgatta ttactgtgca gcatgggatg acagcctgat attcggcgga 300

gggaccaagc tgaccgtcct aggc 324

<210>  20
<211>  108
<212>  PRT
<213>  Homo sapiens

<400>  20

Asn Phe Met Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Asn Asn
            20                  25                  30

Asp Val Tyr Trp Tyr Gln Gln Phe Pro Gly Thr Ala Pro Lys Leu Leu
        35                  40                  45

Ile Tyr Arg Asn Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Ala Ala Trp Asp Asp Ser Leu
                85                  90                  95

Ile Phe Gly Gly Gly Thr Lys Leu Thr Val Leu Gly
            100                 105

<210>  21
<211>  369
<212>  DNA
<213>  Homo sapiens

<400>  21
caggtgcagc tggtggagtc tgggtctgag gtgaagaagc ctggggcctc agtgaaggtc      60

tcctgtaagg cttctggata caccttcacc gactactata tccactgggt gcgacaggcc     120

cctggacaag ggcttgaatg gatgggatgg ctcaacccca atggtggaag tacaaactat     180

ccacagaagt ttcagggctg cgtcaccatg acccgggaca cggccaccag tacagccttc     240

atggagctga gcggcctcaa gtctgacgac acggccttat attactgtgc gagagcgata     300

aaagccgatt cgggcttctc ctaccttctg gacgtctggg gccaagggac cacggtcacc     360

gtctcctca                                                             369

<210>  22
<211>  123
<212>  PRT
<213>  Homo sapiens

<400>  22

Gln Val Gln Leu Val Glu Ser Gly Ser Glu Val Lys Lys Pro Gly Ala
1               5                   10                  15

Ser Val Lys Val Ser Cys Lys Ala Ser Gly Tyr Thr Phe Thr Asp Tyr
            20                  25                  30

Tyr Ile His Trp Val Arg Gln Ala Pro Gly Gln Gly Leu Glu Trp Met
        35                  40                  45

Gly Trp Leu Asn Pro Asn Gly Gly Ser Thr Asn Tyr Pro Gln Lys Phe
    50                  55                  60

Gln Gly Cys Val Thr Met Thr Arg Asp Thr Ala Thr Ser Thr Ala Phe
65                  70                  75                  80

Met Glu Leu Ser Gly Leu Lys Ser Asp Asp Thr Ala Leu Tyr Tyr Cys
                85                  90                  95

Ala Arg Ala Ile Lys Ala Asp Ser Gly Phe Ser Tyr Leu Leu Asp Val
            100                 105                 110

Trp Gly Gln Gly Thr Thr Val Thr Val Ser Ser
        115                 120

<210>  23
<211>  333
<212>  DNA
<213>  Homo sapiens

<400>  23
cagcctgtgc tgactcagcc accctcagcg tctgggaccc ccgggcagag ggtcaccatc      60

tcttgttctg gaagcagctc taacatcgga agtaattatg tatactggta ccagcagctc     120

ccaggaacgg cccccaaact cctcatctat aggattaatc agcggccctc aggggtccgt     180

gaccgattct ctggctccaa gtctggcacc tcagcctccc tggccatcag tgggctccgg     240

tccgaggatg aggctgatta ttactgttca gcatgggatg acagcctgag tggttgggtg     300

ttcggcggag ggaccaagct gaccgtccta ggc                                  333

<210>  24
<211>  111
<212>  PRT
<213>  Homo sapiens

<400>  24

Gln Pro Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Tyr Val Tyr Trp Tyr Gln Gln Leu Pro Gly Thr Ala Pro Lys Leu Leu
        35                  40                  45

Ile Tyr Arg Ile Asn Gln Arg Pro Ser Gly Val Arg Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Ser Ala Trp Asp Asp Ser Leu
                85                  90                  95

Ser Gly Trp Val Phe Gly Gly Gly Thr Lys Leu Thr Val Leu Gly
            100                 105                 110

<210>  25
<211>  360
<212>  DNA
<213>  Homo sapiens

<400>  25
caggtgcagc tgcaggagtc cgggggaggc ttagttcagc ccggagggtc cctgagactc      60

tcctgtacag cctcaggatt cactttcagt aatcactgga tgcactgggt ccgccaaact 120

ccagggaagg ggctggtgtg ggtctcacgt atcagaggtg atgcgggcga cagaaactac 180

gcggactctg tgaagggccg attcaccatc tccagagaca actcccagaa caccctgttt 240

ctgcaaatga acagtctgac agccgaggac acggctgtct attactgtgc aagagatgtg 300

gtccttggtt cgggcagtac tgaccagtgg ggccagggag ccctggtcac cgtctcctca 360

<210> 26
<211> 120
<212> PRT
<213> Homo sapiens

<400> 26

Gln Val Gln Leu Gln Glu Ser Gly Gly Gly Leu Val Gln Pro Gly Gly
1               5                   10                  15

Ser Leu Arg Leu Ser Cys Thr Ala Ser Gly Phe Thr Phe Ser Asn His
            20                  25                  30

Trp Met His Trp Val Arg Gln Thr Pro Gly Lys Gly Leu Val Trp Val
        35                  40                  45

Ser Arg Ile Arg Gly Asp Ala Gly Asp Arg Asn Tyr Ala Asp Ser Val
    50                  55                  60

Lys Gly Arg Phe Thr Ile Ser Arg Asp Asn Ser Gln Asn Thr Leu Phe
65                  70                  75                  80

Leu Gln Met Asn Ser Leu Thr Ala Glu Asp Thr Ala Val Tyr Tyr Cys
                85                  90                  95

Ala Arg Asp Val Val Leu Gly Ser Gly Ser Thr Asp Gln Trp Gly Gln
            100                 105                 110

Gly Ala Leu Val Thr Val Ser Ser
        115                 120

<210> 27
<211> 342
<212> DNA
<213> Homo sapiens

<400> 27
gatattgtga tgacccagac tccagactcc ctggctgtgt ctctgggcga gagggccacc 60

atcaactgca agtccagcca gagtgtttta tacagctcca acagtaagaa ctacttagct 120

tggtaccagc agaaaccagg acagcctcct aagctgctca tttactgggc atctacccgg 180

gaatccgggg tccctgaccg attcagtggc agcgggtctg ggacagattt cactctcacc 240

atcagcagcc tgcaggctga agatgtggca gcttattact gtcagcaata ttctactact 300

ccgttcactt tcggcggagg gaccaagctg gagatcaaag gc 342

<210> 28
<211> 114
<212> PRT
<213> Homo sapiens

<400> 28

Asp Ile Val Met Thr Gln Thr Pro Asp Ser Leu Ala Val Ser Leu Gly
1               5                   10                  15

Glu Arg Ala Thr Ile Asn Cys Lys Ser Ser Gln Ser Val Leu Tyr Ser
            20                  25                  30

Ser Asn Ser Lys Asn Tyr Leu Ala Trp Tyr Gln Gln Lys Pro Gly Gln
        35                  40                  45

Pro Pro Lys Leu Leu Ile Tyr Trp Ala Ser Thr Arg Glu Ser Gly Val
    50                  55                  60

Pro Asp Arg Phe Ser Gly Ser Gly Ser Gly Thr Asp Phe Thr Leu Thr
65                  70                  75                  80

Ile Ser Ser Leu Gln Ala Glu Asp Val Ala Ala Tyr Tyr Cys Gln Gln
                85                  90                  95

Tyr Ser Thr Thr Pro Phe Thr Phe Gly Gly Gly Thr Lys Leu Glu Ile
            100                 105                 110

Lys Gly

<210>  29
<211>  362
<212>  DNA
<213>  Homo sapiens

<400>  29
caggtgcagc tggtggagtc cgggggacgc gtggtccagc ctgggaggtc cctgagcctc      60

tcctgtacaa cctctggatt caccttcagt gactattaca tgcactgggt ccgccaggct     120

ccaggcaagg ggctggagtg ggtggcattt atttcatctg atggaggtaa taagtattat     180

ttagactccg tgaagggccg attcaccgtc tccagagaca attccaagaa cacactatct     240

ctgcaaatga acagcctgaa gactgaggac acggctctgt attactgtgc gggggtgcca     300

gctgttacgt cagcctggta cttcgatctc tggggccgtg gcaccctggt caccgtctct     360

ca                                                                    362

<210>  30
<211>  120
<212>  PRT
<213>  Homo sapiens

<400>  30

Gln Val Gln Leu Val Glu Ser Gly Gly Gly Val Val Gln Pro Gly Arg
1               5                   10                  15

Ser Leu Ser Leu Ser Cys Thr Thr Ser Gly Phe Thr Phe Ser Asp Tyr
            20                  25                  30

Tyr Met His Trp Val Arg Gln Ala Pro Gly Lys Gly Leu Glu Trp Val
        35                  40                  45

Ala Phe Ile Ser Ser Asp Gly Gly Asn Lys Tyr Tyr Leu Asp Ser Val
    50                  55                  60

Lys Gly Arg Phe Thr Val Ser Arg Asp Asn Ser Lys Asn Thr Leu Ser
65                  70                  75                  80

Leu Gln Met Asn Ser Leu Lys Thr Glu Asp Thr Ala Leu Tyr Tyr Cys

85                  90                  95

Ala Gly Val Pro Ala Val Thr Ser Ala Trp Tyr Phe Asp Leu Trp Gly
            100                 105                 110

Arg Gly Thr Leu Val Thr Val Ser
        115                 120

<210>  31
<211>  342
<212>  DNA
<213>  Homo sapiens

<400>  31
gacatccagt tgacccagtc tccagactcc ctggctgtgt ctctgggcga gagggccacc      60

atcaactgca agtccagcca gagtgtttta tacagctcca acaataagaa ctacttagct     120

tggtaccagc agaaaccagg acagcctcct aagctgctca tttactgggc atctacccga     180

gaatctgggg tccctgaccg gttcagtggc agcgggtctg ggacagattt cactctcacc     240

atcagcagcc tgcaggttga agatgtggca gtgtattatt gtcagcaata ttatacttat     300

tccctcactt tcggcggagg gaccaagctg gagatcaaag gc                        342

<210>  32
<211>  114
<212>  PRT
<213>  Homo sapiens

<400>  32

Asp Ile Gln Leu Thr Gln Ser Pro Asp Ser Leu Ala Val Ser Leu Gly
1               5                   10                  15

Glu Arg Ala Thr Ile Asn Cys Lys Ser Ser Gln Ser Val Leu Tyr Ser
            20                  25                  30

Ser Asn Asn Lys Asn Tyr Leu Ala Trp Tyr Gln Gln Lys Pro Gly Gln
        35                  40                  45

Pro Pro Lys Leu Leu Ile Tyr Trp Ala Ser Thr Arg Glu Ser Gly Val
    50                  55                  60

Pro Asp Arg Phe Ser Gly Ser Gly Ser Gly Thr Asp Phe Thr Leu Thr
65                  70                  75                  80

Ile Ser Ser Leu Gln Val Glu Asp Val Ala Val Tyr Tyr Cys Gln Gln
                85                  90                  95

Tyr Tyr Thr Tyr Ser Leu Thr Phe Gly Gly Gly Thr Lys Leu Glu Ile
            100                 105                 110

Lys Gly

<210>  33
<211>  357
<212>  DNA
<213>  Homo sapiens

<400>  33
caggtgacct tgaaggagtc tggtcctgcg ctggtgaaat ccacacagac cctcacgctg      60

acctgcaact tgtctggttt ctctgttaac actagaggag tgggtgtggg ctggctccgt     120

cagcccccag gaaaggccct ggagtggctt gctatgattt attgggatga tgataggcgc 180

tacagtccat acgtgaaaga caggatctcc ctcaccaggg acatctccaa aacccaggtg 240

gtccttacaa tgaccaacat ggagcctgcc gacacaggca catattactg tgcacgcgac 300

tacaatcctc ggtcttttga tcactggggc cagggaaccc tggtcaccgt ctcctca 357

<210> 34
<211> 119
<212> PRT
<213> Homo sapiens

<400> 34

Gln Val Thr Leu Lys Glu Ser Gly Pro Ala Leu Val Lys Ser Thr Gln
1               5                   10                  15

Thr Leu Thr Leu Thr Cys Asn Leu Ser Gly Phe Ser Val Asn Thr Arg
            20                  25                  30

Gly Val Gly Val Gly Trp Leu Arg Gln Pro Pro Gly Lys Ala Leu Glu
        35                  40                  45

Trp Leu Ala Met Ile Tyr Trp Asp Asp Asp Arg Arg Tyr Ser Pro Tyr
    50                  55                  60

Val Lys Asp Arg Ile Ser Leu Thr Arg Asp Ile Ser Lys Thr Gln Val
65                  70                  75                  80

Val Leu Thr Met Thr Asn Met Glu Pro Ala Asp Thr Gly Thr Tyr Tyr
                85                  90                  95

Cys Ala Arg Asp Tyr Asn Pro Arg Ser Phe Asp His Trp Gly Gln Gly
            100                 105                 110

Thr Leu Val Thr Val Ser Ser
        115

<210> 35
<211> 336
<212> DNA
<213> Homo sapiens

<400> 35
aattttatgc tgactcagcc cccctcggcg tctgggaccc ccgggcagac ggtctccatc 60

tcttgttctg gaagcgactc caacatcgga acttatactg ttaactggta ccagcaactc 120

ccaggaacgg cccccaaact cctcatctat agcaatgatc agcggccctc aggggtccct 180

gaccgattct ctggctccaa gtctggcacc tcagcctccc tggccatcag tgggctgcag 240

tccgaggatg aggctgatta tcactgtgca gcatgggatg ccaatttgta tggccatgtg 300

gtattcggcg gagggaccaa ggtggaaatc aaaggc 336

<210> 36
<211> 112
<212> PRT
<213> Homo sapiens

<400> 36

Asn Phe Met Leu Thr Gln Pro Pro Ser Ala Ser Gly Thr Pro Gly Gln
1               5                   10                  15

Thr Val Ser Ile Ser Cys Ser Gly Ser Asp Ser Asn Ile Gly Thr Tyr

            20                  25                  30

Thr Val Asn Trp Tyr Gln Gln Leu Pro Gly Thr Ala Pro Lys Leu Leu
        35                  40                  45

Ile Tyr Ser Asn Asp Gln Arg Pro Ser Gly Val Pro Asp Arg Phe Ser
    50                  55                  60

Gly Ser Lys Ser Gly Thr Ser Ala Ser Leu Ala Ile Ser Gly Leu Gln
65                  70                  75                  80

Ser Glu Asp Glu Ala Asp Tyr His Cys Ala Ala Trp Asp Ala Asn Leu
                85                  90                  95

Tyr Gly His Val Val Phe Gly Gly Gly Thr Lys Val Glu Ile Lys Gly
            100                 105                 110

<210> 37
<211> 357
<212> DNA
<213> Homo sapiens

<400> 37
caggtgcagc tggtggagac tgggggaggc ttggtccagc ctggggggtc cctgagactc 60

tcctgtgcag cctcaggact cagctttagt aactatgcga tgagctgggt ccgccaggct 120

ccaggaaagg ggctggagtg ggtctcaact atcagtaata gtggtgataa cacactctac 180

ccagactccg tgaagggccg gttcaccatc tccagagaca attccagaga cacactatat 240

ctgcacttga gtggcctggg agccgaggac acggccattt actactgtgc gaacgcccca 300

ccatacactc actactttga ccagtggggc cagggaaccc tggtcaccgt ctcctca 357

<210> 38
<211> 119
<212> PRT
<213> Homo sapiens

<400> 38

Gln Val Gln Leu Val Glu Thr Gly Gly Gly Leu Val Gln Pro Gly Gly
1               5                   10                  15

Ser Leu Arg Leu Ser Cys Ala Ala Ser Gly Leu Ser Phe Ser Asn Tyr
            20                  25                  30

Ala Met Ser Trp Val Arg Gln Ala Pro Gly Lys Gly Leu Glu Trp Val
        35                  40                  45

Ser Thr Ile Ser Asn Ser Gly Asp Asn Thr Leu Tyr Pro Asp Ser Val
    50                  55                  60

Lys Gly Arg Phe Thr Ile Ser Arg Asp Asn Ser Arg Asp Thr Leu Tyr
65                  70                  75                  80

Leu His Leu Ser Gly Leu Gly Ala Glu Asp Thr Ala Ile Tyr Tyr Cys
                85                  90                  95

Ala Asn Ala Pro Pro Tyr Thr His Tyr Phe Asp Gln Trp Gly Gln Gly
            100                 105                 110

Thr Leu Val Thr Val Ser Ser
        115

<210> 39
<211> 342
<212> DNA
<213> Homo sapiens

<400> 39
gacatccggt tgacccagtc tccagactcc ctggctgtgt ctctgggcga gagggccacc 60

atcaactgca agtccagtca gagtatttta tacagataca acagtaacaa ctacttagct 120

tggtaccagc agaaaccagg acagcctcct aagttgctca tttactgggc atctacccgg 180

gaatccgggg tccctgaccg attcagtggc agcgggtctg ggacagattt cactctcacc 240

atcagcagtc tgcaggctga agatgtggca gtttattact gtcagcaata ttacagtact 300

cctcagacgt tcggccaagg gaccaaggtg gaaatcaaag gc 342

<210> 40
<211> 114
<212> PRT
<213> Homo sapiens

<400> 40

Asp Ile Arg Leu Thr Gln Ser Pro Asp Ser Leu Ala Val Ser Leu Gly
1               5                   10                  15

Glu Arg Ala Thr Ile Asn Cys Lys Ser Ser Gln Ser Ile Leu Tyr Arg
            20                  25                  30

Tyr Asn Ser Asn Asn Tyr Leu Ala Trp Tyr Gln Gln Lys Pro Gly Gln
        35                  40                  45

Pro Pro Lys Leu Leu Ile Tyr Trp Ala Ser Thr Arg Glu Ser Gly Val
    50                  55                  60

Pro Asp Arg Phe Ser Gly Ser Gly Ser Gly Thr Asp Phe Thr Leu Thr
65                  70                  75                  80

Ile Ser Ser Leu Gln Ala Glu Asp Val Ala Val Tyr Tyr Cys Gln Gln
                85                  90                  95

Tyr Tyr Ser Thr Pro Gln Thr Phe Gly Gln Gly Thr Lys Val Glu Ile
            100                 105                 110

Lys Gly

<210> 41
<211> 366
<212> DNA
<213> Homo sapiens

<400> 41
caggtgcagc tggtgcagtc tgggggaggc ctggtcaagc ctggggggtc cctgagactc 60

tcctgtgcag ccactggctt cagtttcaga cattatggca tgaactgggt ccgccaggcc 120

ccagggaagg ggctggactg ggtctcgtac attagtgata gtagcagcca catctactac 180

gcagactccg tgaggggccg attcagcatc tccagagaca acgccaagga ctcactgttt 240

ctggaagtga acagcctgag acccggggac acggctgctt attactgtgc gagagatacg 300

ggcacctatg cccgcatgtc cggtatggac gtctggggcc aagggaccac ggtcaccgtc 360

tcctca 366

<210>  42
<211>  122
<212>  PRT
<213>  Homo sapiens

<400>  42

Gln Val Gln Leu Val Gln Ser Gly Gly Gly Leu Val Lys Pro Gly Gly
1               5                   10                  15

Ser Leu Arg Leu Ser Cys Ala Ala Thr Gly Phe Ser Phe Arg His Tyr
            20                  25                  30

Gly Met Asn Trp Val Arg Gln Ala Pro Gly Lys Gly Leu Glu Trp Val
        35                  40                  45

Ser Tyr Ile Ser Asp Ser Ser Ser His Ile Tyr Tyr Ala Asp Ser Val
    50                  55                  60

Arg Gly Arg Phe Ser Ile Ser Arg Asp Asn Ala Lys Asp Ser Leu Phe
65                  70                  75                  80

Leu Glu Val Asn Ser Leu Arg Pro Gly Asp Thr Ala Ala Tyr Tyr Cys
                85                  90                  95

Ala Arg Asp Thr Gly Thr Tyr Ala Arg Met Ser Gly Met Asp Val Trp
            100                 105                 110

Gly Gln Gly Thr Thr Val Thr Val Ser Ser
        115                 120

<210>  43
<211>  321
<212>  DNA
<213>  Homo sapiens

<400>  43
gatattgtgc tgacgcagac tccatcctcc ctgtctgcat ctgtaggaga cagagtcacc      60

atcacttgcc gggccagtca gagtattagt gactggttgg cctggtatca gcagaaacca     120

gggaaagccc ctaagctcct gatctataag gcgtctagtt tagaaagtgg ggtcccgtca     180

aggttcagcg gcagtggatc tgggacagag ttcactctca ccatcagcag cctgcagcct     240

gatgattttg caacttatta ctgccaacaa tataatagtt acttcacttt cggccctggg     300

accaaagtgg atatcaaagg c                                               321

<210>  44
<211>  107
<212>  PRT
<213>  Homo sapiens

<400>  44

Asp Ile Val Leu Thr Gln Thr Pro Ser Ser Leu Ser Ala Ser Val Gly
1               5                   10                  15

Asp Arg Val Thr Ile Thr Cys Arg Ala Ser Gln Ser Ile Ser Asp Trp
            20                  25                  30

Leu Ala Trp Tyr Gln Gln Lys Pro Gly Lys Ala Pro Lys Leu Leu Ile
        35                  40                  45

Tyr Lys Ala Ser Ser Leu Glu Ser Gly Val Pro Ser Arg Phe Ser Gly
    50                  55                  60

Ser Gly Ser Gly Thr Glu Phe Thr Leu Thr Ile Ser Ser Leu Gln Pro
65                  70                  75                  80

Asp Asp Phe Ala Thr Tyr Tyr Cys Gln Gln Tyr Asn Ser Tyr Phe Thr
                85                  90                  95

Phe Gly Pro Gly Thr Lys Val Asp Ile Lys Gly
            100                 105

<210>  45
<211>  363
<212>  DNA
<213>  Homo sapiens

<400>  45
caggtgcagc tgcagcagtg gggcgcaggg ctggtgaagc cttcggagac cctgtccctc      60

acctgcgcta tctatggtgg gtccttcagt tcttactact ggaactggat ccgccagccc     120

ccagggaagg gactggagtg gattggggaa atcaatcata gtggaggcac caactacaac     180

ccgtccctca agagtcgagt caccatatca cttgacatgt ccaagaacca cttctccctg     240

aacctgaact ctgtgaccgc cgcggacacg gctgtctatt attgtgcgag agatgctggt     300

aaccgggggt ctacggactg gttcgacccc tggggccagg gaaccctggt caccgtctcc     360

tca                                                                   363

<210>  46
<211>  121
<212>  PRT
<213>  Homo sapiens

<400>  46

Gln Val Gln Leu Gln Gln Trp Gly Ala Gly Leu Val Lys Pro Ser Glu
1               5                   10                  15

Thr Leu Ser Leu Thr Cys Ala Ile Tyr Gly Gly Ser Phe Ser Ser Tyr
            20                  25                  30

Tyr Trp Asn Trp Ile Arg Gln Pro Pro Gly Lys Gly Leu Glu Trp Ile
        35                  40                  45

Gly Glu Ile Asn His Ser Gly Gly Thr Asn Tyr Asn Pro Ser Leu Lys
    50                  55                  60

Ser Arg Val Thr Ile Ser Leu Asp Met Ser Lys Asn His Phe Ser Leu
65                  70                  75                  80

Asn Leu Asn Ser Val Thr Ala Ala Asp Thr Ala Val Tyr Tyr Cys Ala
                85                  90                  95

Arg Asp Ala Gly Asn Arg Gly Ser Thr Asp Trp Phe Asp Pro Trp Gly
            100                 105                 110

Gln Gly Thr Leu Val Thr Val Ser Ser
        115                 120

<210>  47
<211>  324
<212>  DNA

<213>  Homo sapiens

<400>  47
gacatccgga tgacccagtc tccatcctcc ctgtctgcat ctgtaggaga cagagtcacc      60

atcacttgcc gggcaagtca gggcattaga aatgatttag gctggtatca gcagagacca     120

gggaaagccc cgaatctcct gatctatgct gcatccactt tacaaagtgg ggtcccatca     180

agattcagcg gcagtggatc cggcacagat ttcactctca ccatcagcag cctgcagcct     240

gaagattctg caacttatta ctgtctacaa gattacgctt acccacggac gttcggccaa     300

gggaccaagg tggaaatcaa aggc                                            324


<210>  48
<211>  108
<212>  PRT
<213>  Homo sapiens

<400>  48

Asp Ile Arg Met Thr Gln Ser Pro Ser Ser Leu Ser Ala Ser Val Gly
1               5                   10                  15


Asp Arg Val Thr Ile Thr Cys Arg Ala Ser Gln Gly Ile Arg Asn Asp
            20                  25                  30


Leu Gly Trp Tyr Gln Gln Arg Pro Gly Lys Ala Pro Asn Leu Leu Ile
        35                  40                  45


Tyr Ala Ala Ser Thr Leu Gln Ser Gly Val Pro Ser Arg Phe Ser Gly
    50                  55                  60


Ser Gly Ser Gly Thr Asp Phe Thr Leu Thr Ile Ser Ser Leu Gln Pro
65                  70                  75                  80


Glu Asp Ser Ala Thr Tyr Tyr Cys Leu Gln Asp Tyr Ala Tyr Pro Arg
                85                  90                  95


Thr Phe Gly Gln Gly Thr Lys Val Glu Ile Lys Gly
            100                 105


<210>  49
<211>  37
<212>  DNA
<213>  Artificial

<220>
<223>  synthetic primer

<400>  49
agcgcggcct ctagacacgt gcagctgcag gagtcsg                               37


<210>  50
<211>  36
<212>  DNA
<213>  Artificial

<220>
<223>  synthetic primer

<400>  50
agcgcggcct ctagacacgt acagctgcag cagtca                                36


<210>  51
<211>  37
<212>  DNA
<213>  Artificial

<220>
<223>  synthetic primer

<400>  51
agcgcggcct ctagacacgt gcagctacag cagtggg                                37


<210>  52
<211>  36
<212>  DNA
<213>  Artificial

<220>
<223>  synthetic primer

<400>  52
agcgcggcct ctagagacgt gcagctgktg gagwcy                                 36


<210>  53
<211>  38
<212>  DNA
<213>  Artificial

<220>
<223>  synthetic primer

<400>  53
agcgcggcct ctagacacgt ccagctkgtr gagtctgg                               38


<210>  54
<211>  37
<212>  DNA
<213>  Artificial

<220>
<223>  synthetic primer

<400>  54
agcgcggcct ctagacacrt caccttgaag gagtctg                                37


<210>  55
<211>  38
<212>  DNA
<213>  Artificial

<220>
<223>  synthetic primer

<400>  55
agcgcggcct ctagacacgt gcagctggtg sartctgg                               38


<210>  56
<211>  23
<212>  DNA
<213>  Artificial

<220>
<223>  synthetic primer

<400>  56
gacatccrgd tgacccagtc tcc                                               23


<210>  57
<211>  23
<212>  DNA
<213>  Artificial

<220>
<223>  synthetic primer

<400>  57
gaaattgtrw tgacrcagtc tcc                                               23

<210>  58
<211>  23
<212>  DNA
<213>  Artificial

<220>
<223>  synthetic primer

<400>  58
gatattgtgm tgacbcagwc tcc                                              23


<210>  59
<211>  22
<212>  DNA
<213>  Artificial

<220>
<223>  synthetic primer

<400>  59
gaaacgacac tcacgcagtc tc                                               22


<210>  60
<211>  23
<212>  DNA
<213>  Artificial

<220>
<223>  synthetic primer

<400>  60
cagtctgtsb tgacgcagcc gcc                                              23


<210>  61
<211>  22
<212>  DNA
<213>  Artificial

<220>
<223>  synthetic primer

<400>  61
tcctatgwgc tgacwcagcc ac                                               22


<210>  62
<211>  23
<212>  DNA
<213>  Artificial

<220>
<223>  synthetic primer

<400>  62
tcctatgagc tgayrcagcy acc                                              23


<210>  63
<211>  20
<212>  DNA
<213>  Artificial

<220>
<223>  synthetic primer

<400>  63
cagcctgtgc tgactcaryc                                                  20


<210>  64
<211>  23
<212>  DNA
<213>  Artificial

<220>

<223>  synthetic primer

<400>  64
cagdctgtgg tgacycagga gcc                                              23


<210>  65
<211>  23
<212>  DNA
<213>  Artificial

<220>
<223>  synthetic primer

<400>  65
cagccwgkgc tgactcagcc mcc                                              23


<210>  66
<211>  23
<212>  DNA
<213>  Artificial

<220>
<223>  synthetic primer

<400>  66
tcctctgagc tgastcagga scc                                              23


<210>  67
<211>  21
<212>  DNA
<213>  Artificial

<220>
<223>  synthetic primer

<400>  67
cagtctgyyc tgaytcagcc t                                                21


<210>  68
<211>  22
<212>  DNA
<213>  Artificial

<220>
<223>  synthetic primer

<400>  68
aattttatgc tgactcagcc cc                                               22


<210>  69
<211>  25
<212>  DNA
<213>  Artificial

<220>
<223>  synthetic primer

<400>  69
cattgaggag acrgtgacca gggtg                                            25


<210>  70
<211>  25
<212>  DNA
<213>  Artificial

<220>
<223>  synthetic primer

<400>  70
cattgaggag acggtgacca gggtt                                            25


<210>  71

<211>  24
<212>  DNA
<213>  Artificial

<220>
<223>  synthetic primer

<400>  71
cattgaagag acggtgacca ttgt                                              24


<210>  72
<211>  26
<212>  DNA
<213>  Artificial

<220>
<223>  synthetic primer

<400>  72
cattgaggag acggtgaccg tggtcc                                            26


<210>  73
<211>  23
<212>  DNA
<213>  Artificial

<220>
<223>  synthetic primer

<400>  73
catggttggg gcggatgcac tcc                                               23


<210>  74
<211>  24
<212>  DNA
<213>  Artificial

<220>
<223>  synthetic primer

<400>  74
catsgatggg cccttggtgg argc                                              24


<210>  75
<211>  20
<212>  DNA
<213>  Artificial

<220>
<223>  synthetic primer

<400>  75
tttgatttcc accttggtcc                                                   20


<210>  76
<211>  20
<212>  DNA
<213>  Artificial

<220>
<223>  synthetic primer

<400>  76
tttgatctcc ascttggtcc                                                   20


<210>  77
<211>  19
<212>  DNA
<213>  Artificial

<220>
<223>  synthetic primer

<400>  77
ttgatatcca ctttggtcc                                                    19


<210>  78
<211>  20
<212>  DNA
<213>  Artificial

<220>
<223>  synthetic primer

<400>  78
tttaatctcc agtcgtgtcc                                                   20


<210>  79
<211>  20
<212>  DNA
<213>  Artificial

<220>
<223>  synthetic primer

<400>  79
taggacggts ascttggtcc                                                   20


<210>  80
<211>  20
<212>  DNA
<213>  Artificial

<220>
<223>  synthetic primer

<400>  80
gaggacggtc agctgggtgc                                                   20


<210>  81
<211>  22
<212>  DNA
<213>  Artificial

<220>
<223>  synthetic primer

<400>  81
ggacacggcc gtrtattact gt                                                22


<210>  82
<211>  22
<212>  DNA
<213>  Artificial

<220>
<223>  synthetic primer

<400>  82
ggacayggcc atgtattact gt                                                22


<210>  83
<211>  24
<212>  DNA
<213>  Artificial

<220>
<223>  synthetic primer


<220>
<221>  misc_feature
<222>  (9)..(9)
<223>  n is a, c, g, or t

<400>  83

atcagywgns tgsarsctga rgat                                              24


<210>  84
<211>  24
<212>  DNA
<213>  Artificial

<220>
<223>  synthetic primer


<220>
<221>  misc_feature
<222>  (9)..(9)
<223>  n is a, c, g, or t

<400>  84
atcaatwgns tgsarsctga rgat                                              24


<210>  85
<211>  21
<212>  DNA
<213>  Artificial

<220>
<223>  synthetic primer

<400>  85
gaygaggctg actattactg t                                                 21


<210>  86
<211>  21
<212>  DNA
<213>  Artificial

<220>
<223>  synthetic primer

<400>  86
gatgaatctg attattactg t                                                 21


<210>  87
<211>  21
<212>  DNA
<213>  Artificial

<220>
<223>  synthetic primer

<400>  87
gatgaggctg agtatcattg t                                                 21


<210>  88
<211>  21
<212>  DNA
<213>  Artificial

<220>
<223>  synthetic primer

<400>  88
gatgagagtg actaccactg t                                                 21


<210>  89
<211>  22
<212>  DNA
<213>  Artificial

<220>
<223>  synthetic primer

<400>  89
acagtaatay acggccgtgt cc                                                22

<210>  90
<211>  22
<212>  DNA
<213>  Artificial

<220>
<223>  synthetic primer

<400>  90
acagtaatac atggccrtgt cc                                                22


<210>  91
<211>  24
<212>  DNA
<213>  Artificial

<220>
<223>  synthetic primer


<220>
<221>  misc_feature
<222>  (16)..(16)
<223>  n is a, c, g, or t

<400>  91
atcytcagsy tscasncwrc tgat                                              24


<210>  92
<211>  24
<212>  DNA
<213>  Artificial

<220>
<223>  synthetic primer


<220>
<221>  misc_feature
<222>  (16)..(16)
<223>  n is a, c, g, or t

<400>  92
atcytcagsy tscasncwat tgat                                              24


<210>  93
<211>  21
<212>  DNA
<213>  Artificial

<220>
<223>  synthetic primer

<400>  93
acagtaatag tcagcctcrt c                                                 21


<210>  94
<211>  21
<212>  DNA
<213>  Artificial

<220>
<223>  synthetic primer

<400>  94
acagtaataa tcagattcat c                                                 21


<210>  95
<211>  21
<212>  DNA
<213>  Artificial

<220>
<223>  synthetic primer

<400>  95
acaatgatac tcagcctcat c                                                21


<210>  96
<211>  21
<212>  DNA
<213>  Artificial

<220>
<223>  synthetic primer

<400>  96
acagtggtag tcactctcat c                                                21


<210>  97
<211>  29
<212>  DNA
<213>  Artificial

<220>
<223>  synthetic primer

<400>  97
tgtgatggct tccatgtcgg cagaatgct                                        29


<210>  98
<211>  31
<212>  DNA
<213>  Artificial

<220>
<223>  synthetic primer

<400>  98
gctaaacaac tttcaacagt ctatgcggca c                                     31


<210>  99
<211>  8
<212>  PRT
<213>  Artificial

<220>
<223>  Synthetic peptide

<400>  99

Asp Ile Ser Lys Ala His Asp Tyr
1               5


<210>  100
<211>  22
<212>  PRT
<213>  Artificial

<220>
<223>  Synthetic peptide

<400>  100

Gly Gly Gly Lys Glu Val Thr Val Leu Gly Leu Pro Val Gly Trp Asp
1               5                   10                  15


Tyr Tyr Gly Met Asp Ile
            20


<210>  101
<211>  8
<212>  PRT
<213>  Artificial

<220>
<223>  Synthetic peptide

<400>  101

Gln Arg Trp Ser Asn Phe Asp Tyr
1               5


<210>  102
<211>  13
<212>  PRT
<213>  Artificial

<220>
<223>  Synthetic peptide

<400>  102

Ser Gly Leu Tyr Gly Thr Thr Pro Ser Thr Phe Asp His
1               5                   10


<210>  103
<211>  13
<212>  PRT
<213>  Artificial

<220>
<223>  Synthetic peptide

<400>  103

Ala Asp Pro Gly Tyr Met Ile Phe Asn Trp Phe Asp Pro
1               5                   10


<210>  104
<211>  13
<212>  PRT
<213>  Artificial

<220>
<223>  Synthetic peptide

<400>  104

Ala Ile Lys Ala Asp Ser Gly Phe Tyr Leu Leu Asp Val
1               5                   10


<210>  105
<211>  11
<212>  PRT
<213>  Artificial

<220>
<223>  Synthetic peptide

<400>  105

Asp Val Val Leu Gly Ser Gly Ser Thr Asp Gln
1               5                   10


<210>  106
<211>  21
<212>  PRT
<213>  Artificial

<220>
<223>  Synthetic peptide

<400>  106

Gln Phe Gln Glu His Thr Ile Ser Ala Asn Glu Gln Pro Glu Asp Gly
1               5                   10                  15

His Gly Ser Val Leu
            20

<210>  107
<211>  9
<212>  PRT
<213>  Artificial

<220>
<223>  Synthetic peptide

<400>  107

Asp Tyr Asn Pro Arg Ser Phe Asp His
1               5

<210>  108
<211>  11
<212>  PRT
<213>  Artificial

<220>
<223>  Synthetic peptide

<400>  108

Asn Ala Pro Pro Tyr Thr His Tyr Phe Asp Gln
1               5                   10

<210>  109
<211>  13
<212>  PRT
<213>  Artificial

<220>
<223>  Synthetic peptide

<400>  109

Asp Thr Gly Thr Tyr Ala Arg Met Ser Gly Met Asp Val
1               5                   10

<210>  110
<211>  13
<212>  PRT
<213>  Artificial

<220>
<223>  Synthetic peptide

<400>  110

Asp Ala Gly Asn Arg Gly Ser Tyr Asp Trp Phe Asp Pro
1               5                   10

## Description

### Technical Field

The present invention relates to phage-displayed single chain antibodies which recognize non-reducing mannose residues. More specifically, the present invention relates to single chain antibodies recognizing non-reducing mannose residues which are obtained by screening of phage-display antibody library.

### Background Art

As generally known, immunization with carbohydrates often leads to a primary IgM response and no response in some cases because many carbohydrates are self-antigens (Non-patent Documents 1 and 2). Phage display technology, which allows generation of antibodies against self-antigens, is thus considered the best available strategy to produce antibodies directed against carbohydrate moieties. The possibility of isolating antibody fragments with high specificity and affinity for carbohydrates by phage display technique is appealing since conventional hybridoma technologies have proven to be ineffective in producing monoclonal antibodies directed against a variety of carbohydrate moieties. Phage display technology has been used mostly to generate antibodies against proteins, whereas its use for carbohydrate antigens has been rarely done. Because of difficulties in immobilization of carbohydrate antigens onto plastic plates, the same procedures generally used for protein antigens cannot be directly applied to carbohydrate antigens. Previous studies thus utilized glycoproteins, heteroglycans, and carbohydrate-BSA conjugates as antigens to produce anti-carbohydrate single chain antibodies (scFvs) by phage display methods (Non-patent Documents 1, and 3 to 7). Since the majority of anti-carbohydrate antibodies generated so far belong to the IgM class if they are monoclonal antibodies, or have low affinity if they are scFvs, they are not suitable for *in vivo* diagnostics or therapy. Low affinity to carbohydrate antigens is most likely due to the intrinsic nature of carbohydrates, which may not be easily overcome. Attempts have been made to overcome the low-affinity nature of anti-carbohydrate antibodies by increasing the antibody titer (Non-patent Documents 7 to 10). It has been shown that multivalent scFv have higher affinity to carbohydrate moieties (Non-patent Documents 9 to 10).
[Non-patent Document 1] Deng, S., MacKenzie, C.R., Sadowaka, J., Michniewicz, J., Young, N.M., Bundle, D.R., and Narang, S.A. (1994) J. Biol. Chem. 269, 9533-9538.
[Non-patent Document 2] MacKenzie, C.R., Hirama. T., Deng, S., Bundle, D.R., Narang, S.A., and Young, N.M. (1996) J. Biol. Chem. 271, 1527-1533.
[Non-patent Document 3] Babino, A., Pritsch, O., Oppezzo, P., Du Pasquier, R., Roseto, A., Osinaga, E., and Alzari, P.M. (1997) Hybridoma 16, 317-324.
[Non-patent Document 4] van Kuppevelt, T.H., Dennissen, M.A.B.A., van Venrooij, W.J., Hoet, R.M.A., and Veerkamp, J.H. (1998) J. Biol. Chem. 273, 12960-12966.
[Non-patent Document 5] Mao, S., Gao, C., Lo, C.-H. L., Wirsching, P., Wong, C.-H., and Janda K.D. (1999) Proc. Natl. Acad. Sci. USA 96, 6953-6958.
[Non-patent Document 6] Lee, K.J., Mao. S., Sun, C., Gao, C., Blixt, O., Arrues, S., Hom. L.G., Kaufmann, G.F., Hoffman, T.Z., Coyle, A.R., Paulson, J., Felding-Habermann, B., and Janda, K.D. (2002) J. Am. Chem. Soc. 124, 12439-12446.
[Non-patent Document 7] Ravn, P., Danielczyk, A., Jensen, K.B., Kristensen, P., Christensen, P.A., Larsen, M., Karsten, U., and Goletz, S. (2004) J. Mol. Biol. 343, 985-996
[Non-patent Document 8] Deng, S., MacKenzie, C.R., Hirama, T., Brousseau, R., Lowary, T.L., Young, N.M., Bundle, D.R., and Narang, S.A. (1995) Proc. Natl. Acad. Sci. USA 92, 4992-4996.
[Non-patent Document 9] MacKenzie, R. and To, R. (1998) J. Immunol. Methods 220, 39-49.
[Non-patent Document 10] Zhang, J., Tanha, J., Hirama, T., To, N.H.K.R., Tong-Sevinc, H., Stone, E., Brisson, J.-R., and MacKenzie, C.R. (2004) .l. Mol. Biol. 345, 49-56.

### Disclosure of the Invention

### Object to be Solved by the Invention

It is an object of the present invention to provide antibodies which recognize non-reducing mannose residues, and nucleic acids encoding the antibodies.

### Means for Solving the Object

The inventors of the present invention have adapted phage-display technology to generate human scFvs using artificial glycolipids as antigens (Stoll, M. S., Mizuochi, T., Childs, R. A., and Feizi, T. (1988) J. Biochem. 256, 661-665; Kenjo, A., Takahashi, M., Matsushita, M., Endo, Y., Nakata, M., Mizuochi, T., and Fujita, T. (2001) J. Biol. Chem. 276, 19959-19965; and Shimizu, Y., Nakata, M., Matsunuma, J., and Mizuochi, T. (2001) J. Chromatogr. B. Biomed. Sci. Appl. 754, 127-133). It is possible to isolate candidate phage antibodies directed against various carbohydrate antigens by immobilizing soluble carbohydrate antigens via lipid anchors on plastic titer-plates, and subsequent panning and screening by ELISA. To achieve the above object, 30 or more types of artificial glycolipids were synthesized in this invention.

To establish methodologies to build up a set of scFvs directed against various carbohydrate moieties, the inventors of the present invention have screened phage-display library representing 10¹¹ or more types of human scFvs (Sblattero, D. and Bradbury, A. (2000) Nat. Biotechnol. 18, 76-80). The present invention used, as a model antigens, Man3-DPPE which is an artificial glycolipid synthesized from mannotriose (Man3) and dipalmitoylphosphatidylethanolamine (DPPE) by reductive amination according to previous reports (Stoll, M. S., Mizuochi, T., Childs, R. A., and Feizi, T. (1988) J. Biochem. 256, 661-665; Kenjo, A., Takahashi, M., Matsushita, M., Endo, Y., Nakata, M., Mizuochi, T., and Fujita, T. (2001) J. Biol. Chem. 276, 19959-19965; and Shimizu, Y., Nakata, M., Matsunuma, J., and Mizuochi, T. (2001) J. Chromatogr. B. Biomed. Sci. Appl. 754, 127-133). Characterization of positive phage clones by TLC-overlay assays and surface plasmon resonance analyses revealed that these phage antibody clones bound to artificial glycolipids bearing mannose residues at nonreducing termini. In addition, binding of the phage antibodies to RNase B carrying high mannose type oligosaccharides but not to fetuin carrying complex type and O-linked oligosaccharides was observed. Furthermore, first round characterization of scFvs produced from respective phages indicated good affinity and specificity for nonreducing mannose residues. Thus, it was demonstrated the validity of a phage display technology in constructing human scFv against various carbohydrate antigens.

Thus the present invention provides an antibody of any of the following (1) to (14):
(1) an antibody recognizing a non-reducing mannose residue, comprising
   the amino acid sequence of SEQ ID NO: 2 or an amino acid sequence, wherein one or a few amino acids have been deleted, substituted, or added in the amino acid sequence of SEQ ID NO: 2, as an amino acid sequence of heavy chain variable region, and the amino acid sequence of SEQ ID NO: 4 or an amino acid sequence, wherein one or a few amino acids have been deleted, substituted, or added in the amino acid sequence of SEQ ID NO: 4, as an amino acid sequence of light chain variable region;
(2) an antibody recognizing a non-reducing mannose residue, comprising
   the amino acid sequence of SEQ ID NO: 6 or an amino acid sequence, wherein one or a few amino acids have been deleted, substituted, or added in the amino acid sequence of SEQ ID NO: 6, as an amino acid sequence of heavy chain variable region, and the amino acid sequence of SEQ ID NO: 8 or an amino acid sequence, wherein one or a few amino acids have been deleted, substituted, or added in the amino acid sequence of SEQ ID NO: 8, as an amino acid sequence of light chain variable region;
(3) an antibody recognizing a non-reducing mannose residue, comprising
   the amino acid sequence of SEQ ID NO: 10 or an amino acid sequence, wherein one or a few amino acids have been deleted, substituted, or added in the amino acid sequence of SEQ ID NO: 10, as an amino acid sequence of heavy chain variable region, and the amino acid sequence of SEQ ID NO: 12 or an amino acid sequence, wherein one or a few amino acids have been deleted, substituted, or added in the amino acid sequence of SEQ ID NO: 12, as an amino acid sequence of light chain variable region;
(4) an antibody recognizing a non-reducing mannose residue, comprising
   the amino acid sequence of SEQ ID NO: 14 or an amino acid sequence, wherein one or a few amino acids have been deleted, substituted, or added in the amino acid sequence of SEQ ID NO: 14, as an amino acid sequence of heavy chain variable region, and the amino acid sequence of SEQ ID NO: 16 or an amino acid sequence, wherein one or a few amino acids have been deleted, substituted, or added in the amino acid sequence of SEQ ID NO: 16, as an amino acid sequence of light chain variable region;
(5) an antibody recognizing a non-reducing mannose residue, comprising
   the amino acid sequence of SEQ ID NO: 18 or an amino acid sequence, wherein one or a few amino acids have been deleted, substituted, or added in the amino acid sequence of SEQ ID NO: 18, as an amino acid sequence of heavy chain variable region, and the amino acid sequence of SEQ ID NO: 20 or an amino acid sequence, wherein one or a few amino acids have been deleted, substituted, or added in the amino acid sequence of SEQ ID NO: 20, as an amino acid sequence of light chain variable region;
(6) an antibody recognizing a non-reducing mannose residue, comprising
   the amino acid sequence of SEQ ID NO: 22 or an amino acid sequence, wherein one or a few amino acids have been deleted, substituted, or added in the amino acid sequence of SEQ ID NO: 22, as an amino acid sequence of heavy chain variable region, and the amino acid sequence of SEQ ID NO: 24 or an amino acid sequence, wherein one or a few amino acids have been deleted, substituted, or added in the amino acid sequence of SEQ ID NO: 24, as an amino acid sequence of light chain variable region;
(7) an antibody recognizing a non-reducing mannose residue, comprising
   the amino acid sequence of SEQ ID NO: 26 or an amino acid sequence, wherein one or a few amino acids have been deleted, substituted, or added in the amino acid sequence of SEQ ID NO: 26, as an amino acid sequence of heavy chain variable region, and the amino acid sequence of SEQ ID NO: 28 or an amino acid sequence, wherein one or a few amino acids have been deleted, substituted, or added in the amino acid sequence of SEQ ID NO: 28, as an amino acid sequence of light chain variable region;
(8) an antibody recognizing a non-reducing mannose residue, comprising
   the amino acid sequence of SEQ ID NO: 30 or an amino acid sequence, wherein one or a few amino acids have been deleted, substituted, or added in the amino acid sequence of SEQ ID NO: 30, as an amino acid sequence of heavy chain variable region, and the amino acid sequence of SEQ ID NO: 32 or an amino acid sequence, wherein one or a few amino acids have been deleted, substituted, or added in the amino acid sequence of SEQ ID NO: 32, as an amino acid sequence of light chain variable region;
(9) an antibody recognizing a non-reducing mannose residue, comprising
   the amino acid sequence of SEQ ID NO: 34 or an amino acid sequence, wherein one or a few amino acids have been deleted, substituted, or added in the amino acid sequence of SEQ ID NO: 34, as an amino acid sequence of heavy chain variable region, and the amino acid sequence of SEQ ID NO: 36 or an amino acid sequence, wherein one or a few amino acids have been deleted, substituted, or added in the amino acid sequence of SEQ ID NO: 36, as an amino acid sequence of light chain variable region;
(10) an antibody recognizing a non-reducing mannose residue, comprising
   the amino acid sequence of SEQ ID NO: 38 or an amino acid sequence, wherein one or a few amino acids have been deleted, substituted, or added in the amino acid sequence of SEQ ID NO: 38, as an amino acid sequence of heavy chain variable region, and the amino acid sequence of SEQ ID NO: 40 or an amino acid sequence, wherein one or a few amino acids have been deleted, substituted, or added in the amino acid sequence of SEQ ID: 40, as an amino acid sequence of light chain variable region;
(11) an antibody recognizing a non-reducing mannose residue, comprising
   the amino acid sequence of SEQ ID NO: 42 or an amino acid sequence, wherein one or a few amino acids have been deleted, substituted, or added in the amino acid sequence of SEQ ID NO: 42, as an amino acid sequence of heavy chain variable region, and the amino acid sequence of SEQ ID NO: 44 or an amino acid sequence, wherein one or a few amino acids have been deleted, substituted, or added in the amino acid sequence of SEQ ID: 44, as an amino acid sequence of light chain variable region; and
(12) an antibody recognizing a non-reducing mannose residue, comprising
   the amino acid sequence of SEQ ID NO: 46 or an amino acid sequence, wherein one or a few amino acids have been deleted, substituted, or added in the amino acid sequence of SEQ ID NO: 46, as an amino acid sequence of heavy chain variable region, and the amino acid sequence of SEQ ID NO: 48 or an amino acid sequence, wherein one or a few amino acids have been deleted, substituted, or added in the amino acid sequence of SEQ ID NO: 48, as an amino acid sequence of light chain variable region.

Another aspect of the present invention provides a nucleic acid encoding the aforementioned antibody of the present invention.

Further another aspect of the present invention provides a nucleic acid of any of the following:
(1) a nucleic acid comprising the nucleotide sequence of SEQ ID NO: 1 and the nucleotide sequence of SEQ ID NO: 3;
(2) a nucleic acid comprising the nucleotide sequence of SEQ ID NO: 5 and the nucleotide sequence of SEQ ID NO: 7;
(3) a nucleic acid comprising the nucleotide sequence of SEQ ID NO: 9 and the nucleotide sequence of SEQ ID NO: 11;
(4) a nucleic acid comprising the nucleotide sequence of SEQ ID NO: 13 and the nucleotide sequence of SEQ ID NO: 15;
(5) a nucleic acid comprising the nucleotide sequence of SEQ ID NO: 17 and the nucleotide sequence of SEQ ID NO: 19;
(6) a nucleic acid comprising the nucleotide sequence of SEQ ID NO: 21 and the nucleotide sequence of SEQ ID NO: 23;
(7) a nucleic acid comprising the nucleotide sequence of SEQ ID NO: 25 and the nucleotide sequence of SEQ ID NO: 27;
(8) a nucleic acid comprising the nucleotide sequence of SEQ ID NO: 29 and the nucleotide sequence of SEQ ID NO: 31;
(9) a nucleic acid comprising the nucleotide sequence of SEQ ID NO: 33 and the nucleotide sequence of SEQ ID NO: 35;
(10) a nucleic acid comprising the nucleotide sequence of SEQ ID NO: 37 and the nucleotide sequence of SEQ ID NO: 39;
(11) a nucleic acid comprising the nucleotide sequence of SEQ ID NO: 41 and the nucleotide sequence of SEQ ID NO: 43; and
(12) a nucleic acid comprising the nucleotide sequence of SEQ ID NO: 45 and the nucleotide sequence of SEQ ID NO: 47.

Further another aspect of the present invention provides a process for producing an antibody wherein the aforementioned nucleic acid of the present invention is used.

Further another aspect of the present invention provides a recombinant antibody recognizing a nonreducing mannose residue which can be obtained by the aforementioned process. Preferably, the antibody is a single chain antibody.

### Best Mode for Carrying Out the Invention

Hereafter, embodiments of the present invention will be more specifically described.

The antibodies of the present invention are antibodies which recognize non-reducing mannose residues. As described in the Examples below, the antibodies of the present invention have been obtained through screening of phage-display library of human scFvs, with use of Man3-DPPE, as a model antigen, which is an artificial glycolipid synthesized from mannotriose (Man3) and dipalmitoylphosphatidylethanolamine (DPPE) by reductive amination. As a result of characterization of positive phage clones by TLC-overlay assays and surface plasmon resonance analyses, it was demonstrated that the antibodies of the present invention bound to artificial glycolipids bearing mannose residues at nonreducing termini.

The glycolipid for use as an antigen in screening of the present invention is not specifically limited, and any artificial glycolipid bearing a mannose residue at a nonreducing terminal may be used, although oligosaccharides are preferred.

In an oligosaccharide, individual constituent sugars bind to one another via an α→1,2 bond, an α→1,3 bond, an α→1,4 bond, an α→1,6 bond, a β→1,4 bond, or a combination thereof. For example, mannose may constitute a straight chain through the abovementioned bond, or may adopt a branched structure via the combination of an α→1,3 bond with an α→1,6 bond. The number of monosaccharides in an oligosaccharide is preferably 2 to 11.

Specific examples of such an oligosaccharide include mannobiose (Man2), mannotriose (Man3), mannotetraose (Man4), mannopentaose (Man5), mannohexaose (Man6), and mannoheptaose (Man7).

These oligosaccharides have a reducing terminal aldehyde group. Thus, such an aldehyde group can be used as a means for immobilizing an oligosaccharide. That is to say, an aldehyde group is allowed to react with a lipid having an amino group to form a Schiff base. Subsequently, according to a common method, the Schiff base is reduced, and is preferably chemically reduced by NaBH₃CN, for example, so as to bind the oligosaccharide to the lipid.

The aforementioned lipid having an amino group is preferably a phospholipid having an amino group. For example, phosphatidylamine such as dipalmitoylphosphatidylethanolamine (DPPE) or distearoylphosphatidylethanolamine (DSPE) can be used. Thus obtained bound substance of an oligosaccharide and a lipid may be referred to as an artificial glycolipid in the present invention. The glycolipid for use in the present invention is preferably an artificial glycolipid as mentioned above.

The solvent for dissolving an artificial glycolipid serving as an antigen is not specifically limited, although it is preferable to dissolve such an artificial glycolipid in a solvent system of chloroform/methanol/water (10:10:3, v/v) and subsequently dilute it with methanol at 40 µg/ml, for example. This solution is added in, for example, a 96-well plastic plate (flat bottom) and dried in air so as to immobilize the artificial glycolipid onto the wells. These wells are blocked and washed. The plate coated with an artificial glycolipid by such an operation can be used as an antigen-coated plate.

Tris-buffered saline (hereunder, referred to as TBS) is desirably used as the buffer solution. In addition, for example, TBS containing 3% bovine serum albumin (hereunder, referred to as 3% BSA/TBS) is desirable as the blocking solution, and TBS containing 0.2% Tween 20 (hereunder, referred to as TBS-T) is desirable as the washing solution.

The phage-displayed antibodies can be produced by publicly known methods (Marks JD et al. (1991) J. Mol. Biol., 222, 581-597; Nissim A. et al. (1994) EMBO J., 13, 692-698*;* TAKAYANAGI Atsushi, OKUI Michiyo, and SHIMIZU, Nobuyoshi, ARTIFICIAL ANTIBODY LIBRARY WITH SUPER-REPERTORY, Japanese Patent Application No. 2001-358602 (PCT International Publication No. WO 03/04419)).

The phage-display antibody library is constructed by adapting a system in which antibodies are fused with the coat protein of a filamentous phage to thereby display these antibodies on the phage surface. Specifically, such a phage-display library can be made by PCR amplification of antibody genes to produce a library including many types of antibody genes, and displaying them on a phage. Hereunder, specific example of the method for producing phage-displayed single chain antibodies is described, but is not to be considered as limiting.

Human peripheral blood cDNA library and human spleen cDNA library are used as templates to amplify a fragment including CDR1 and CDR2 regions in VH or VL region of immunoglobulin gene, and a fragment including CDR3 region by PCR. These fragments are mixed and used as templates to amplify the VH or VL region by PCR. These amplified DNA fragments are respectively inserted into a non-expressing phagemid vector to produce a VH library and a VL library. These *E. coli* VH library and VL library are infected with helper phages to convert into phage libraries. These phage-type VH library and VL library are coinfected into *E. coli* expressing Cre-recombinase, to effect recombination between the VH and VL vectors within *E. coli.* This *E. coli* strain is infected with helper phages M13KO7, by which phages expressing full length single-chain antibodies can be produced. To remove unrecombinants mixed in these phages, *E. coli* is infected with these phages while avoiding multiple infection, then superinfected with helper phages, and incubated in a medium which contains ampicillin/ kanamycin/chloramphenicol but does not contain glucose at 25°C. By so doing, phages expressing recombinant single-chain antibodies can be obtained in the supernatant. The phages in the supernatant are precipitated with polyethylene glycol and resuspended, by which the artificial antibody library with a large repertoire (10¹¹ or more clones) can be obtained.

Further, a phage-displayed antibody having a binding property to a carbohydrate of interest can be selectively collected from the human single-chain phage library produced by the above manner, with reference to the binding property to an artificial glycolipid having the concerned carbohydrate structure. This operation may be referred to as panning.

The carbohydrate-specific binding property of phage-displayed antibodies obtained by repetition of panning is desirably analyzed by the ELISA method that will be described later, for example, because a large number of phage clone samples can be simultaneously analyzed. That is to say, a carbohydrate is immobilized onto a 96-well plastic plate and blocked with a buffer solution containing 3% BSA for example, in order to avoid non-specific adsorption of phage-displayed antibodies. A suspension of the phage-displayed antibodies which had been adjusted at an appropriate concentration with a buffer solution is added thereto, followed by sufficient reaction (for example, at 37°C for 2 hours), and subsequent washing with a buffer solution.

To detect/quantify phage-displayed antibodies bound to the carbohydrate, antibodies are reacted with an aqueous solution containing anti-phage antibodies that have been labeled with a substance suitable for detection, followed by washing with a buffer solution, and subsequent detection/quantification in an optimum method for detecting the labeling substance. At this time, there is no specific limitation in the labeling substance for the anti-phage antibodies, although labeling with horseradish peroxidase is desirable for example, in terms of handiness. In addition, there is no specific limitation in the detector for use in the detection, although a plate reader suitable for ELISA is desirably employed.

The term "antibody" used in the present invention does not only refer to antibodies in a form normally existing *in vivo*, but also include peptides containing at least one antigen-binding site formed of the variable region in the H chain or L chain of the antibody, or a combination thereof, Fab composed of a set of an H chain fragment and an L chain fragment, F(ab')₂ composed of two sets of H chain fragments and L chain fragments, single chain antibodies composed of an H chain fragment and an L chain fragment bound in series in a single peptide (hereunder, may be referred to as "scFvs"), and the like. The "antibody" of the present invention may be a full length antibody in a form normally existing in vivo, which is composed of two sets of full length H chains and full length L chains.

The terms "F(ab')₂" and "Fab" in the present invention mean antibody fragments produced by treatment of an immunoglobulin with a protease such as pepsin or papain, and generated by digestion around the disulfide bonds existing between two H chains in the hinge region. For example, when IgG1 is treated with papain, cleavages occur in upstreams of the disulfide bonds existing between two H chains in the hinge region to allow the production of two identical antibody fragments in which an L chain composed of VL (L chain variable region) and CL (L chain constant region) and an H chain fragment composed of VH (H chain variable region) and CHγ1 (γ1 region within H chain constant region) are connected by a disulfide bond at the C-terminal region. These two identical antibody fragments are respectively referred to as Fab'. In addition, when IgG is treated with pepsin, cleavages occur in downstreams of the disulfide bonds existing between two H chains in the hinge region to allow the production of an antibody fragment which is slightly larger than the combined product having said two Fab's connected by the hinge region. This antibody fragment is referred to as F(ab')₂.

Usually, an antibody consists of two types of large and small polypeptides. The large subunit is referred to as "H chain (heavy chain)" and the small subunit is referred to as "L chain (light chain)". In addition, each peptide is composed of a "variable region" existing at the N-terminal side and forming an antigen-binding site, and a "constant region" which is conserved per each antibody class. The variable region is further divided into complementarity determining regions "CDRs" which particularly involve the formation of the antigen-binding site, and "framework regions" existing therebetween. CDRs are known consist of three regions called "CDR1", "CDR2", and "CDR3" from the N-terminal side, for each H chain and L chain.

The single chain antibodies of the present invention can be prepared by appropriately selecting inducible vectors such as pSE380 plasmid (Invitrogen) or pET24d(+) plasmid (Novagen) and host bacterial cells. In addition to the above method, in the production of the antibodies of the present invention, an animal cell expression system, an insect cell expression system, and a yeast cell expression system can also be used. The linker for linking the H chain and the L chain can also be appropriately selected by those skilled in the art.

SEQ ID NOS: 1 to 48 in the Sequence Listing as shown below represent the nucleotide sequences and the amino acid sequences of H chains and L chains of the antibodies of the present invention. Amino acid sequences in which one or a few amino acids (the number of amino acids is preferably 1 to 8, more preferably 1 to 5, and yet more preferably 1 to 3) have been deleted, substituted, or added in the aforementioned amino acid sequences are also included in the scope of the present invention as long as an antibody recognizing a non-reducing mannose residue of a glycolipid can be provided. Nucleic acids comprising the nucleotide sequences represented in the Sequence Listing shown below can be obtained by either the procedure described in Examples of the present application, or chemical synthesis with use of a DNA synthesizer. In addition, nucleotide sequences encoding the amino acid sequences in which one or a few amino acids have been deleted, substituted, or added in the amino acid sequences in the Sequence Listing shown below, can also be readily obtained through usual gene recombination technique such as PCR, or chemical synthesis with use of a DNA synthesizer, by those skilled in the art.
SEQ ID NO:1:Nucleotide sequence of variable region of heavy chain of antibody M3-2
SEQ ID NO:2: Amino acid sequence of variable region of heavy chain of antibody M3-2
SEQ ID ND:3:Nucleotide sequence of variable region of light chain of antibody M3-2
SEQ ID NO:4: Amino acid sequence of variable region of light chain of antibody M3-2
SEQ ID NO:5:Nucleotide sequence of variable region of heavy chain of antibody M3-4
SEQ ID NO:6: Amino acid sequence of variable region of heavy chain of antibody M3-4
SEQ ID NO:7:Nucleotide sequence of variable region of light chain of antibody M3-4
SEQ ID NO:8: Amino acid sequence of variable region of light chain of antibody M3-4
SEQ ID NO:9:Nucleotide sequence of variable region of heavy chain of antibody M3-5
SEQ ID NO:10:Amino acid sequence of variable region of heavy chain of antibody M3-5
SEQ ID NO:11:Nucleotide sequence of variable region of light chain of antibody M3-5
SEQ ID NO:12:Amino acid sequence of variable region of light chain of antibody M3-5
SEQ ID NO:13:Nucleotide sequence of variable region of heavy chain of antibody M3-6
SEQ ID NO:14:Amino acid sequence of variable region of heavy chain of antibody M3-6
SEQ ID NO:15:Nucleotide sequence of variable region of light chain of antibody M3-6
SEQ ID NO:16:Amino acid sequence of variable region of light chain of antibody M3-6
SEQ ID NO:17:Nuelcotide sequence of variable region of heavy chain of antibody M3-7
SEQ ID NO:18:Amino acid sequence of variable region of heavy chain of antibody M3-7
SEQ ID NO:19:Nucleotide sequence of variable region of light chain of antibody M3-7
SEQ ID NO:20:Amino acid sequence of variable region of light chain of antibody M3-7
SEQ ID NO:21:Nucleotide sequence of variable region of heavy chain of antibody M3-8
SEQ ID NO:22:Amino acid sequence of variable region of heavy chain of antibody M3-8
SEQ ID NO:23:Nucleotide sequence of variable region of light chain of antibody M3-8
SEQ ID NO:24:Amino acid sequence of variable region of light chain of antibody M3-8
SEQ ID NO:25:Nucleotide sequence of variable region of heavy chain of antibody M3-9
SEQ ID NO:26:Amino acid sequence of variable region of heavy chain of antibody M3-9
SEQ ID NO:27:Nucleotide sequence of variable region of light chain of antibody M3-9
SEQ ID NO:28:Amino acid sequence of variable region of light chain of antibody M3-9
SEQ ID NO:29:Nucleotide sequence of variable region of heavy chain of antibody M3-10
SEQ ID NO:30:Amino acid sequence of variable region of heavy chain of antibody M3-10
SEQ ID NO:31:Nucleotide sequence of variable region of light chain of antibody M3-10
SEQ ID NO:32:Amino acid sequence of variable region of light chain of antibody M3-10
SEQ ID NO:33:Nucleotide sequence of variable region of heavy chain of antibody M3-11
SEQ ID NO:34:Amino acid sequence of variable region of heavy chain of antibody M3-11
SEQ ID NO:35:Nucleotide sequence of variable region of light chain of antibody M3-11
SEQ ID NO:36:Amino acid sequence of variable region of light chain of antibody M3-11
SEQ ID NO:37:Nucleotide sequence of variable region of heavy chain of antibody M3-12
SEQ ID NO:38:Amino acid sequence of variable region of heavy chain of antibody M3-12
SEQ ID NO:39:Nucleotide sequence of variable region of light chain of antibody M3-12
SEQ ID NO:40:Amino acid sequence of variable region of light chain of antibody M3-12
SEQ ID NO:41:Nucleotide sequence of variable region of heavy chain of antibody M3-13
SEQ ID NO:42:Amino acid sequence of variable region of heavy chain of antibody M3-13
SEQ ID NO:43:Nucleotide sequence of variable region of light chain of antibody M3-13
SEQ ID NO:44:Amino acid sequence of variable region of light chain of antibody M3-13
SEQ ID NO:45:Nucleotide sequence of variable region of heavy chain of antibody M3-14
SEQ ID NO:46:Amino acid sequence of variable region of heavy chain of antibody M3-14
SEQ ID ND:47:Nucleotide sequence of variable region of light chain of antibody M3-14
SEQ ID NO:48:Amino acid sequence of variable region of light chain of antibody M3-14

Further, an antibody in a form normally existing in vivo can be prepared from scFv. For example, only the variable regions of the H chain and the L chain are amplified by PCR from a scFv plasmid. Each fragment is, for example, recombined into a plasmid having the H chain gene and/or the L chain gene of a human antibody, which thereby enables the formation of an antibody having a variable region on the scFv in a form normally existing *in vivo*. Specifically, for example, appropriate restriction enzyme cleavage sites are introduced at both ends of the gene fragment obtained when amplifying the variable regions of the H chain and the L chain from the plasmid, and they are combined with an appropriate restriction enzyme cleavage site on the plasmid having the H chain and/or the L chain of the human antibody, thereby replacing genes in the variable region without causing frame-shift. Thus, an antibody in a form normally existing *in vivo* which has a sequence of a variable region on a plasmid as it is, can be prepared. Further, a peptide containing at least one antigen-binding site formed of the variable region of the H chain or L chain of the antibody, or a combination thereof, Fab composed of a set of an H chain fragment and an L chain fragment, and (Fab'2) composed of two sets of H chain fragments and L chain fragments can also be prepared from the antibody.

The expression of the antibody of the present invention can be carried out by employing E. *coli,* yeast, insect cells, animal cells, and the like. For example, when an antibody is expressed in COS cell or CHO cell, pCDNA3.1(+) or pMAMneo (CLONETECH) can be used. For example, a gene of the H chain of the antibody obtained in the above method is incorporated into a multicloning site of pCDNA3.1(+), and a gene of the L chain is incorporated into pMAMneo. Then, an expression unit having a gene of the L chain between a promoter and poly A is incorporated into an adequate site of the vector having the H chain incorporated therein. Introduction of this vector into a COS cell, a CHO-K1, or a CHO DG44 by a conventional genetic engineering technique enables the production of the antibody of interest. Further, the expression unit of the DHFR gene is cleaved out from for example, pSV2/DHFR (Nature, 1981. Vol. 294, Lee F. *et al*.) into the above prepared vector, and is incorporated into a vector which expresses the H chain and the L chain. This vector is introduced into the CHO DG44 by a conventional genetic engineering technique. Thus selected cells can be used to significantly improve the productivity of antibodies by utilizing the DHFR gene amplification system using MTX.

Animal cells such as COS cell or CHO cell can be generally cultured in Dulbecco's Modified Eagle's Medium (DMEM) supplemented with 10% fetal bovine serum (FBS) under 5% CO₂ at 37°C. A method for introducing a gene into the COS cell may be electroporation, as well as a DEAE-dextran method and a method using a transfection reagent such as lipofectin.

At the time of production of the antibody of the present invention, the cells are preferably cultured in a serum-free medium in order to prevent the contamination of a serum-derived bovine antibody. COS and CHO cells which are not acclimatized in a serum-free medium but are cultured in serum media, are preferably cultured in serum-free DMEM. The antibody of the present invention, which is thus obtained in the culture supernatant, can be easily purified by a conventional method for purifying IgG antibodies using, for example, Protein A column and Protein G column.

The present invention will be more specifically described in the following examples. However, these Examples are not intended to limit the scope of the present invention. Abbreviations used in this application are as follows: Fv, antibody variable region; scFv, single-chain antibody variable region; Ab, antibody; mAb, monoclonal antibody; BSA, bovine serum albumin; IGF, insulin-like growth factor ; Man1, mannose; Man2, mannobiose (Manα1-6Man); Man3, mannotriose [Manα1-6(Manα1-3)Man]; Man5, mannopentaose [Manα1-6(Manα1-3)Manα1-6(Manα1-3)Man]; GN2Man3, GlcNAcβ1-2Manα1-6(GlcNAcβ1-2Manα1-3)Man; GN2, di-N-acetylchitobiose (GlcNAcβ1-4GlcNAc); FG, Fucα1-2Gal; LNT, lacto-N-tetraose (Galβ1-3GlcNAcβ1-3Galβ1-4Glc); and HRP, horseradish peroxidase.

### Examples

### (A) Materials and Methods

### (1) Materials

Escherichia coli strains used were the suppressor mutant strain TG1 and the nonsuppressor mutant strain TDP10F' from Invitrogen (Carlsbad, CA). Helper phages M13KO7 were obtained from Amersham Biotech (UK). Bovine serum albumin (BSA), fetuin, asialo-fetuin, RNase A, RNase B, DPPE, and dipalmitoylphosphatidylcholine (DPPC) were purchased from Sigma-Aldrich (St. Louis, MO). Mannose (Man1), mannobiose (Man2; Manα1-6Man), mannotriose (Man3; Manα1-6(Manα1-3)Man), mannopentaose (Man5; Manα1-6(Manα1-3)Manα1-6(Manα1-3)Man), di-N-acetylchitobiose (GN2; GlcNAcβ1-4GlcNAc), lacto-N-tetraose (LNT; Galβ1-3GlcNAcβ1-3Galβ1-4Glc), Fucα1-2Gal(FG), GlcNAcβ1-2Manα1-6(GlcNAcβ1-2Manα1-3)Man(GN2Man3), Man1-BSA, and Man3-BSA were obtained from Dextra Laboratories (Reading, UK). Glucose-sp-biotin, Man3-sp-biotin, and Lewis b-sp-Biotin were obtained from GlycoTech (Gaithersburg, MD). ABTS/H₂O₂ was obtained from Roche Diagnostics (Mannheim, Germany). Oxalic acid was obtained from Wako Pure Chemical (Osaka, Japan). Anti-M13 antibodies were purchased obtained from Amersham Bio-Science (Piscataway, NJ) and Exalpha Biologicals (Watertown, MA). Anti-insulin-like growth factor (IGF)-I receptor scFv phage antibody was prepared from 1H7 mAb-derived scFv (Li, S.-L., Liang, S.-J., Guo, N., Wu, A. M., Fujita-Yamaguchi, Y. (2000) Cancer Immunol. Immunother. 49, 243-252) using Expression Module/Recombinant Phage Antibody System of Amersham Bio-Science.

### (2) Preparation of Artificial glycolipids

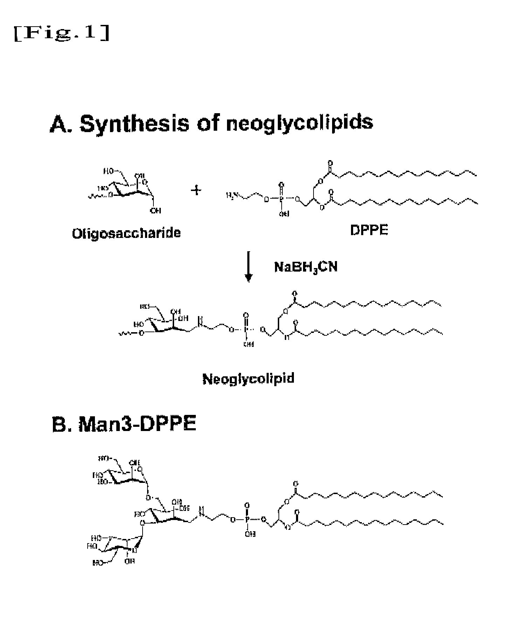

Artificial glycolipids were synthesized from carbohydrates (Man1, Man2, Man3, Man5, GN2, LNT, FG, or GN2Man3) and DPPE by reductive amination according to previous methods (Stoll, M. S., Mizuochi, T., Childs, R. A., and Feizi, T. (1988) J. Biochem. 256, 661-665; Kenjo, A., Takahashi, M., Matsushita, M., Endo, Y., Nakata, M., Mizuochi, T., and Fujita, T. (2001) J. Biol. Chem. 276, 19959-19965; and Shimizu, Y., Nakata, M., Matsunuma, J., and Mizuochi, T. (2001) J. Chromatogr. B. Biomed. Sci. Appl. 754, 127-133). The reaction scheme is shown in Fig. 1A. Briefly, 940 µl of chloroform/methanol (1:1, v/v) solution containing 9.4 mg of DPPE and 200 µl of methanol solution containing 4 mg of sodium cyanoborohydride were added and mixed to 60 µl of aqueous solution containing each carbohydrate (1-2 mg). The reaction was carried out at 80°C for 5 hours with occasional sonication. The artificial glycolipids in the reaction mixtures were then purified by HPLC on a silica gel column Shim-pack PREP-SIL (Shimadzu, Kyoto, Japan) and reversed-phase chromatography on a Bond Elut C18 cartridge column (Varian, Harbor City, CA) according to a previous method (Shimizu, Y., Nakata, M., Matsunuma, J., and Mizuochi, T. (2001) J. Chromatogr. B. Biomed. Sci. Appl. 754, 127-133). The purity and structure of each artificial glycolipid were confirmed with TLC and MALDI-TOF mass spectrometry.

### (3) Construction of Phage Library

### cDNA, PCR Amplification, and Cloning of VH and VL Gene Repertoire To Construct Primary Phage Libraries

PCR templates used in this study were human spleen cDNA purchased from BioChain (Hayward, CA) and human leukocyte cDNA purchased from BD Biosciences (Palo Alto, CA). The human immunoglobulin variable regions were amplified by PCR using an equimolar mixture of appropriate forward and reverse primers having sequences in common with this gene family, according to previously published information (Sbalattero, D. & Bradbury, A. (1998) Immunotechnology 3, 271-278).

5' -primer:
- VHf1: AGCGCGGCCtctagaCAGGTGCAGCTGCAGGAGTCSG(SEQ ID NO:49)
- VHf2: AGCGCGGCCtctagaCAGGTACAGCTGCAGCAGTCA(SEQ ID NO:50)
- VHf3: AGCGCGGCCtctagaCAGGTGCAGCTACAGCAGTGGG(SEQ ID NO:51)
- VHf4: AGCGCGGCCtctagaGAGGTGCAGCTGKTGGAGWCY(SEQ ID NO:52)
- VHf5: AGCGCGGCCtctagaCAGGTCCAGCTKGTRGAGTCTGG(SEQ ID NO:53)
- VHf6: AGCGCGGCCtctagaCAGRTCACCTTGAAGGAGTCTG(SEQ ID NO:54)
- VHf7: AGCGCGGCCtctagaCAGGTGCAGCTGGTGSARTCTGG(SEQ ID NO:55)
- Vkf1: GACATCCRGDTGACCCAGTCTCC(SEQ ID NO:56)
- Vkf2: GAAATTGTRWTGACRCAGTCTCC(SEQ ID NO:57)
- Vkf3: GATATTGTGMTGACBCAGWCTCC(SEQ ID NO:58)
- Vkf4: GAAACGACACTCACGCAGTCTC(SEQ ID NO:59)
- Vlf1: CAGTCTGTSBTGACGCAGCCGCC(SEQ ID NO:60)
- Vlf2: TCCTATGWGCTGACWCAGCCAC(SEQ ID NO:61)

- Vlf3: TCCTATGAGCTGAYRCAGCYACCtSEQ ID NO:62)
- Vlf4: CAGCCTGTGCTGACTCARYC(SEQ ID N0:63)
- Vlf5: CAGDCTGTGGTGACYCAGGAGCC(SEQ ID N0:64)
- VIf6: CAGCCWGKGCTGACTCAGCCMCC(SEQ ID N0:65)
- Vlf7: TCCTCTGAGCTGASTCAGGASCC(SEQ ID N0:66)
- Vlf8: CAGTCTGYYCTGAYTCAGCCT(SEQ ID N0:67)
- Vlf9: AATTTTATGCTGACTCAGCCCC(SEQ ID NO:68)

3' -primer:
- VHr1: CATTGAGGAGACRGTGACCAGGGTG(SEQ ID N0:69)
- VHr2: CATTGAGGAGACGGTGACCAGGGTT(SEQ ID NO:70)
- VHr3: CATTGAAGAGACGGTGACCATTGT(SEQ ID NO:71)
- VHr4: CATTGAGGAGACGGTGACCGTGGTCC(SEQ ID N0:72)
- VHr5: CATGGTTGGGGCGGATGCACTCC(SEQ ID NO:73)
- VHr6: CATSGATGGGCCCTTGGTGGARGC(SEQ ID NO:74)
- VKr1: TTTGATTTCCACCTTGGTCC(SEQ ID NO:75)
- VKr2: TTTGATCTCCASCTTGGTCC(SEQ ID NO:76)
- VKr3: TTGATATCCACTTTGGTCC(SEQ ID NO:77)
- VKr4: TTTAATCTCCAGTCGTGTCC(SEQ ID NO:78)
- V1r1: TAGGACGGTSASCTTGGTCC(SEQ ID NO:79)
- V1r2: GAGGACGGTCAGCTGGGTGC(SEQ ID NO:80)

Primer for CDR shuffling
- VH(FWR)f1: GGACACGGCCGTRTATTACTGT(SEQ ID NO:81)
- VH(FWR)f2: GGACAYGGCCATGTATTACTGT(SEQ ID NO:82)
- Vk(FWR)f1: ATCAGYWGNSTGSARSCTGARGAT(SEQ ID N0:83)
- Vk(FWR)f2: ATCAATWGNSTGSARSCTGARGAT(SEQ ID N0:84)
- V1(FWR)f1: GAYGAGGCTGACTATTACTGT(SEQ ID N0:85)
- V1(FWR)f2: GATGAATCTGATTATTACTGT(SEQ ID N0:86)
- V1(FWR)f3: GATGAGGCTGAGTATCATTGT(SEQ ID N0:87)
- V1(FWR)f4: GATGAGAGTGACTACCACTGT(SEQ ID N0:88)
- VH(FWR)r1: ACAGTAATAYACGGCCGTGTCC(SEQ ID NO:89)
- VH(FWR)r2: ACAGTAATACATGGCCRTGTCC(SEQ ID NO:90)
- Vk(FWR)r1: ATCYTCAGSYTSCASNCWRCTGAT(SEQ ID NO:91)
- Vk(FWR)r2: ATCYTCAGSYTSCASNCWATTGAT(SEQ ID NO:92)
- Vl(FWR)r1: ACAGTAATAGTCAGCCTCRTC(SEQ ID NO:93)
- Vl(FWR)r2: ACAGTAATAATCAGATTCATC(SEQ ID NO:94)
- Vl(FWR)r3: ACAATGATACTCAGCCTCATC(SEQ ID NO:95)
- Vl(FWR)r4: ACAGTGGTAGTCACTCTCATC(SEQ ID NO:96)

Reaction mixtures (50 µl) contained 0.5 µl of the cDNA solution, 25 pmol of each of forward primer and reverse primer, 200 µM dNTPs, 10 mM KCI, 10 mM (NH₄)₂SO₄, 20 mM Tris-HCl (pH 8.8), 2 mM MaCl₂, 100 µg/ml BSA, and 1 unit of KOD plus DNA polymerase (Toyobo, Osaka, Japan). The PCR condition was optimized for each primer set. The first round PCR products purified by agarose gel electrophoresis were mixed in the same quantity as used for templates in the following PCR to amplify immunoglobulin variable regions. The resulting DNA fragments of the variable regions were purified and digested with appropriate restriction enzymes followed by insertion into pVH-Hyg or pVL-Amp vectors. After ligation, the resulting phagemid DNA was electroporated into *E. coli* DH10B strain harboring F' plasmid derived from *E. coli* XL1-Blue, and colonies were grown at 30°C on agar plates containing 1% glucose and hygromycin (50 µg/ml) or ampicillin (50 µg/ml). The colonies were collected and converted to phages bearing VH or VL genes by superinfection with helper phage M13KO7 in 2x YT medium containing 1% glucose at 30°C overnight. The phages were collected by centrifugation and precipitation with polyethylene glycol (PEG) according to a standard protocol.

### Recombination and Generation of Secondary Phage scFv Library

The F' plasmid derived from *E. coli* XL1-Blue strain was introduced into *E. coli* NS3529 strain, which expresses Cre-recombinase constitutively, to obtain *E. coli* NS3529/F' strain. The NS3529/F' strain was infected with VH and VL phages at MOI = 50, and was left still at 37°C for 1 hour without shaking, and was then superinfected with M13KO7 helper phages at MOI = 20. After samples were incubated at 37°C for 1 hour, ampicillin and kanamycin were added at 50 µg/ml and 25 µg/ml, respectively. During overnight culture at 37°C, Cre/lox recombination resulted in formation of scFv genes because of insertion of a VH fragment into the pVL-Amp vector in the NS3529/F' cells. Growing XL1-Blue strain was infected with the recombinant phages obtained from the culture at MOI < 0.1, and was left still at 37°C for 1 hour. ScFv displaying phages fused with a coat protein g3p were obtained from the strain which was cultured in 2x YT medium (glucose free) containing 20 µg/ml chloramphenicol, 50 µg/ml ampicillin and 25 µg/ml kanamycin at 25°C overnight. The phages which were concentrated by PEG precipitation were suspended in SM buffer containing 1% gelatin and 6% DMSO, and were stored at -80°C.

### (4) Phage Selection Methods

### Panning Procedures

The library was subjected to four rounds of panning. Fifty and sixteen wells of a 96-well plate were coated with Man3-DPPE (2 µg/well) and used for first and second panning, respectively. Five and two wells coated with Man3-DPPE (2 µg/well) were used for third and fourth panning, respectively. Phage selection was basically carried out according to previously published procedures (Marks, J.D., Hoogenboom, H.R., Bonnet, T.P., McCafferty, J., Griffiths, A.D., and Winter, G. (1991) J. Mol. Biol. 222, 581-597) with some modifications. Coating of wells with Man3-DPPE is described briefly. Wells are applied with 50 µl of Man3-DPPE (40 µg/ml methanol solution) and the solvent is dried at 37°C, followed by incubation with 150 µl of Tris-HCI buffer (pH 7.4) containing 0.15 M NaCl and 3% BSA (blocking buffer) at 4°C overnight. The wells were rinsed twice with 50 µl of 0.2% Tween 20/TBS (TBS-T), and once with 200 µl of TBS. 50 µl of phage suspension in TBS containing 0.1% Tween 20 and 1.4% BSA was added to the wells, which were then incubated at 37°C for 60 minutes under shaking. After the wells were washed 3 times with 200 µl of TBS-T and twice with 200 µl of TBS, bound phages were eluted by addition of 50 µl of 100 mM triethylamine and incubation at 25°C for 10 minutes. Then, the resultant product was neutralized by mixing with 100 µl of neutralizing solution (1 M Tris-HCl (pH 7.4):3% BSA/TBS = 2:1, v/v) in wells of the control plate, which had been treated with the blocking buffer. Bound phages were further eluted by addition of 50 µl of 100 mM triethylamine and incubation at 25°C for 20 minutes and were recovered in the neutralization solution as described above. Eluted phages were used to infect 100 µl of logarithmic growing *E. coli* TGl strain at 37°C for 1 hour. Infected bacteria were grown on LB agar plates (medium) (diameter = 10 cm) containing 1 mM NaOH, 0.1% glucose, and carbenicillin (50 µg/ml) at 25°C for 16 hours, and then were collected from the plates using a spreader after addition of 2 ml of LB-10 mM Tris-HCl (pH 7.5) (SBS) per plate. 1 ml out of the 38 ml of this suspension was inoculated into 40 ml of SBS containing carbenicillin and grown with shaking at 37°C for 2 hours. 40 µl of solution containing 3.5 x 10⁹ pfu. of helper phages was added and the resultant was incubated at 37°C for 1 hour without shaking. Then, kanamycin (25 µg/ml)/ chloramphenicol (10 µg/ml) was added, and the resultant was incubated with rotation at 25°C for 40 hours. Thus, phages were collected. Phage particles were concentrated by PEG-precipitation, and were dissolved in 400 µl of TBS, 400 µl of 3% BSA/TBS, and 40 ml of 10% Tween 20/TBS by incubation at 37°C for 1 hour, After centrifugation (at 18,000g at 4°C for 5 minutes), 800 µl of phage suspension were recovered and used for second panning. Second, third, and fourth panning were carried out similarly to first panning except for washing conditions. Bacteria picked from single colonies after the fourth panning were grown in 50 µl of SBS/carbenicillin in 96 well plates at 37°C for 1 hour with rotation, to which 50 µl of helper phages were added and incubated at 37°C for 1 hour with rotation. After SBS/kanamycin/chloramphenicol mixture was added at 50 µl/well, phage suspensions were collected through incubation at 25°C for 16 hours with rotation and centrifugation (at 200 g at 4°C for 15 minutes). 50 µl of the supernatants were added to wells containing 100 µl of 3% BSA/TBS, incubated at 37°C for 1 hour, and kept at 4°C.

### Screening of Phage Clones Expressing Antibodies Directed against Man3-DPPE (by ELISA)

Analysis of binding of phages to Man3-DPPE was performed by ELISA using bacterial supernatants containing phages. A 96-well plate was coated with Man3-DPPE (1 µg/well) as described above and blocked by incubation with 150 µl of 3% BSA/TBS at 4°C overnight. Control plates were prepared as above without antigen. 75 µl of phage suspensions were added to the wells and incubated at 37°C for 1 hour. The wells were washed 5 times with 200 µl of TBS-T. Bound phage antibodies were detected by incubation with horseradish peroxidase (HRP)-labeled anti-M13 antibody at 37°C for 1 hour, after which they were washed 10 times with 200 µl of TBS-T and once with 200 µl of TBS. Peroxidase activity was detected by reaction with ABTS/H₂O₂ for 30 minutes and termination with 1% oxalic acid. Then, the absorbance was measured with a BIO-RAD plate-reader at 415 nm.

### (5) Characterization of Phage Antibodies

### Colony PCR and Determination of DNA Sequences

scFv genes were amplified from respective *E. coli* TGl colonies infected with phages by PCR with a primer set (forward primer Cm-f: 5'-TGTGATGGCTTCCATGTCGGCAGAATGCT-3' (SEQ ID NO: 97), and reverse primer g3-r: 5'-GCTAAACAACTTTCAACAGTCTATGCGGCAC-3' (SEQ ID NO: 98)). After preheating the sample at 94°C for 2 minutes, PCR was carried out with 35 cycles under conditions of denaturing at 94°C for 20 seconds, annealing at 53°C for 20 seconds, and extension reaction at 68°C for 1 minute. After purification and confirmation of the length of the PCR product in 2% agarose gel electrophoresis, the resulting scFv genes were subjected to DNA sequencing. DNA sequences of scFvs were determined using a 3730 DNA analyzer (Applied Biosystem, Foster City, CA).

### Binding Assays on TLC Plates

400 pmol of artificial glycolipids dissolved in chloroform/methanol/water (50:55:18, v/v) were dot-blotted onto TLC plates. Alternatively, mixtures containing 1 µg each of artificial glycolipids were spotted on aluminum-backed silica gel 60 high-performance TLC plates (10 cm-length; Merck, Darmstadt, Germany), and were developed with a solvent system of chloroform/methanol/water (60:35:8, v/v). After drying, these plates were soaked for 30 seconds in n-hexane containing 0.1% (w/v) Plexigum P28 (Sigma-Aldrich) and then blocked with 3% BSA/TBS at room temperature for 1 hour followed by washing with TBS. The plates were then overlaid with phage antibody (10¹³ cfu/ml) at 4°C overnight followed by overlay with mouse anti-M13 phage coat protein (p8) IgG (5000-fold dilution) at room temperature for 1 hour. The binding of phage antibodies to artificial glycolipids was detected by a combination of HRP-labeled anti-mouse IgG (MBL, Nagoya, Japan) and a chemiluminescent reagent (ECL^{™} Western blotting detection reagents, Amersham Bio-Sciences, Buckinghamshire, UK) according to the manufacturer's instructions.

### Determination of Carbohydrate Specificity of Phage Antibodies through Examination of their Reactivity to Natural Glycoproteins

Fetuin, asialo-fetuin, RNase A, and RNase B (5 µg/lane) were separated by SDS-polyacrylamide gel electrophoresis (10% gel) and blotted onto a PVDF membrane. The SDS-PAGE gel was stained with Coomassie Brilliant Blue. The reactivity of phage antibody to natural glycoproteins was examined by Western blotting with M3-7 phage antibody followed by HRP-labeled anti-M13 phage antibody as described above.

### Expression and Partial Purification of scFv Proteins

Each isolated phage clone was subjected to infection to *E. coli* TOP10F' strain containing chaperon/repressor vector. The TOP10F' cells infected with M3-7 or a control anti-FLAG phage were cultured in 200 ml of 2x YT medium containing 50 µg/ml ampicillin, 50 µg/ml spectinomycin and 1 mM isopropyl-thio β-D-galactopyranoside (IPTG) at 30°C for 3 hours. Supernatants, periplasmic fractions, or whole cell extracts of the TOP10F' cells were collected by differential centrifugation. Whole cell extracts, collected from 40 ml of the culture solution, were poured into 1 ml of 50 mM Tris-HCI (pH 8) containing 6 M guanidine-HCI, 5% glycerol, 0.5 mM PMSF, and 0.1 mM DTT, and kept on ice for 1 hour for solubilization. After centrifugation (at 18,800 g for 30 minutes), the supernatants were dialyzed against 10 mM HEPES (pH 7.4) containing 150 mM NaCl. Aggregates were removed by centrifugation and then the supernatants were subjected to SDS-PAGE/Western blotting analysis. A total protein amount of 18 µg/lane was separated by SDS-PAGE (4-20% acrylamide gel) (Daiichi Pure Chemicals, Tokyo, Japan) under reducing conditions and then transferred to a PVDF membrane. After blocking with 3% BSA/PBS, scFv proteins expressed were detected with an HRP-labeled anti-E-Tag antibody (Amersham Bio-Sciences, Japan). The scFv protein samples were used for SPR analysis.

### Binding of scFv proteins to Synthetic Glycoproteins (by ELISA)

scFv proteins were produced by the following method for use in the specificity assay to various BSA-conjugated oligosaccharides. TOP10F'-FS2 cells were infected with Man3-specific phages, and cultured in 5 ml of SBS with 1% glucose, 50 µg/ml carbenicillin, and 50 µg/ml spectinomycin at 25°C for 16 hours. Next, the infected cells were collected by centrifugation, then suspended, and cultured in 40 ml of SBS with carbenicillin and spectinomycin at 30°C for 3 hours. After 1 mM IPTG was added, the suspension was cultured at 30°C for 16 hours to induce scFv proteins, and the supernatant was collected. After dot-blotting onto a nitrocellulose membrane, HRP-labeled anti-E-tag antibody was added to confirm the expression of scFv proteins.

The ELISA plate was coated with 50 µl of BSA-conjugated oligosaccharide (10 µl/ml) at 4°C for 16 hours. The supernatant of the culture solution was added to the wells (50 µl/well) and left at 37°C for 2 hour. Binding of scFv proteins to oligosaccharides was assayed according to the above method with use of HRP-labeled anti-E-tag antibody.

### Characterization of Phage Antibodies and scFv Proteins (Surface Plasmon Resonance, SPR)

SPR analyses were performed at 25°C. Solutions were freshly prepared, degassed, and filtered through 0.22-µm pore filter. Binding property of phage antibodies were determined by SPR using BIAcore X (Pharmacia Biosensor). Used in this analysis were HPA sensor chips where aliphatic chains were covalently bound to a gold surface, and 10 mM HEPES (pH 7.4) containing 150 mM NaCl (HBS) as a running buffer. Immobilization of artificial glycolipids on the HPA sensor chip surface was carried out according to the manufacturer's protocol. Briefly, a lipid monolayer with carbohydrates on the surface was formed on the HPA sensor chip by adding artificial glycolipids bearing liposomes. Before immobilization, the surface was cleaned by injection of nonionic detergent, 40 mM MEGA9 (Dojindo, Kumamoto, Japan) and 50% ethanol at a flow rate of 5 µl/min. DPPC or Man3-DPPE/DPPC (1:10, mol/mol) was then applied to the sensor chip surface at a low flow rate of 2 µl/min. To remove multilamellar structures from the lipid surface, 5 µl of 10 mM NaOH was injected at a flow rate of 5 µl/min. Phage antibody samples that had been dialyzed against HBS were allowed to flow for 10 minutes over the surface of the chips at a flow rate of 5 µl/min. To examine the competitive inhibitory effect on SPR, phage antibody samples were mixed with 10 mM or 100 mM D-mannose or α-methyl-D-mannopyranoside (Nacalai Tesque, Kyoto, Japan), and then incubated at 4°C for 1 hour.

SPR analyses of scFv protein samples were performed at a flow rate of 5 µl/min with BIAcore 3000 using the Sensor chip HPA or the Sensor chip SA (Streptavidin). Immobilization of DPPC, Man3-DPPE/DPPC (1:10, mol/mol), Man2-DPPE/DPPC (1:10, mol/mol), Man5-DPPE/DPPC (1:10, mol/mol) to HPA sensor chips was performed as described above. The amounts of immobilized ligands were calculated from the molar ratio of the DPPE conjugates included in the lipid layer, which indicated that DPPC, Man2-DPPE, Man3-DPPE, and Man5-DPPE were 0.43 pmol, 0.31 pmol, 0.48 pmol, and 0.30 pmol, respectively. HBS containing 0.01% Tween 20 (HBS-T) was used as a running buffer when SA sensor chips were used. SA sensor chip surface was activated with three consecutive 1-min injections of activating buffer (1 M NaCl containing 50 mM NaOH) prior to immobilization of biotinylated ligands. Glucose-sp-Biotin, Man3-sp-Biotin, and Lewis b-sp-Biotin (0.1 mM) were diluted with HBS-T to 0.05 µM, 10 µl of which were injected manually at a flow rate of 1 µl/min until RU of 100-150 is achieved. Unlike artificial glycolipids used above, these ligands contained a spacer (sp) consisting of a structure of -O(CH₂)₃NHCO(CH₂)₅NH-. The amounts of immobilized biotinylated carbohydrates were estimated to be 0.25 pmol, 0.23 pmol, and 0.13 pmol, respectively. The scFv samples that had been dialyzed against HBS-T were injected at a flow rate of 1 µl/min for 4 minutes over the surface of the chips. After one sample was assayed, the sensor chip surfaces were regenerated by treating with 10 mM glycine-HCl (pH 1.5) for 1 minute, followed by washing with a continuous flow of HBS-T before the next sample was injected.

### (B) Results

### (1) Screening of scFv-Displaying Phages Directed against Man3-DPPE

The phage displayed human scFv library was subjected to four rounds of panning against Man3-DPPE. The structure of Man3-DPPE is presented in Fig. 1B. Man3-DPPE (2 µg/well) was used for panning according to abovementioned methods. Of 672 clones screened by ELISA using Man3-DPPE (1 µg/well) as an antigen, 40 or more clones was ELISA-positive with S/N of>2. Of those, 25 positive clones encoding scFvs were selected as candidates for phage antibodies directed against the Man3 structure. DNA sequencing of scFv regions of those phage clones revealed that all the clones analyzed had different sequences. Characteristics of 12 clones are shown in Table 1.

**Table 1: Man3-Specific Single Chain Antibodies**

| Clones | Sequences of CDR3 regions of VH Chains | V_{H} family | V_{L} family |
|---|---|---|---|
| M3-2 | DISKAHDY | V_{H}II | VκIII |
| M3-4 | GGGKEVTVLGLPVGWDYYGMDI | V_{H}IV | VκI |
| M3-5 | ORWSNFDY | V_{H}II | VκIII |
| M3-6 | SGLYGTTPSTFDH | V_{H}IV | VλI |
| M3-7 | ADPGYMIFNWFDP | V_{H}IV | VλI |
| M3-8 | AIKADSGFYLLDV | V_{H}I | VλI |
| M3-9 | DVVLGSGSTDQ | V_{H}III | VκIV |
| M3-10 | QFQEHTISANEQPEDGHGSVL | V_{H}III | VκIV |
| M3-11 | DYNPRSFDH | V_{H}II | VλI |
| M3-12 | NAPPYTHYFDQ | V_{H}III | VκIV |
| M3-13 | DTGTYARMSGMDV | V_{H}III | VκI |
| M3-14 | DAGNRGSYDWFDP | V_{H}IV | VκI |

### (2) Carbohydrate Specificity of Phage Clones

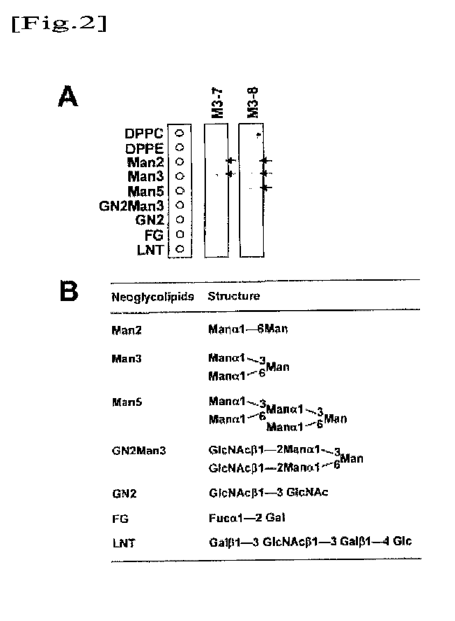

In order to quickly identify which phage antibodies have specificity and high affinity for the antigen used, it is necessary to establish methods suitable for characterizing scfv-presenting phages as phage antibodies. Although expression, isolation, and characterization of scFv proteins are ultimately required, expression and isolation of scFv proteins are labor-intensive and time-consuming, and yet primary information on phage clones is essential to select candidate clones for further characterization. For this purpose, a set of artificial glycolipids was individually spotted on TLC plates (Fig. 2A), and then phage antibodies were overlaid on the plates to allow their binding to the artificial glycolipids. These experiments revealed that phage antibodies showed specificity to Man3, as well as to Man2 and Man5 to a lesser extent as compared to Man3 (Fig. 2A). In addition, since binding to GN2Man3 and DPPE was not observed, it was also demonstrated that the phage antibodies examined bound to non-reducing mannose residues but not to the lipid portion of artificial glycolipids. The structures of artificial glycolipids used in these experiments are illustrated in Fig. 2B.

### (3) Binding of Phage Antibodies to Natural Glycoproteins

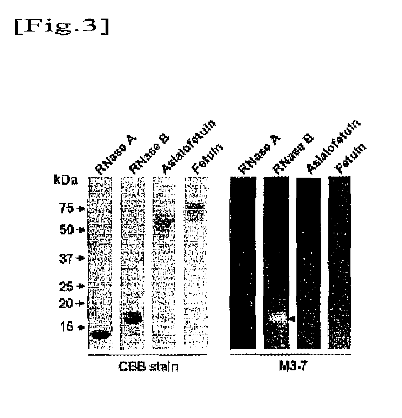

Phage antibodies which were isolated by using Man3-DPPE as a target antigen were shown to have specificity to non-reducing mannose residues of artificial glycolipids. Therefore, it was next examined whether these phage antibodies can bind to the non-reducing mannose residues of glycoproteins. It was clearly shown that M3-7 phage antibody bound to RNase B carrying high mannose type oligosaccharides but not to non-glycosylated RNase A or to fetuin carrying complex type and O-linked oligosaccharides (Fig. 3).

### (4) SPR Analyses of Man3-Specific scFv-Displaying Phage Antibodies

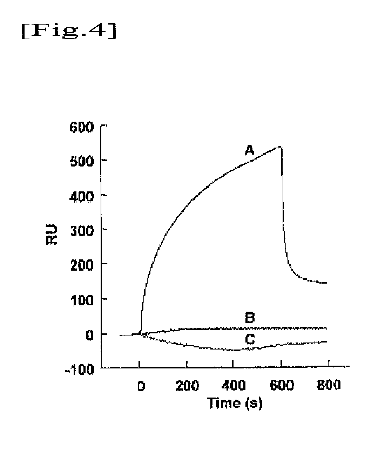

After specificity of phage antibodies to Man3 was confirmed by ELISA and TLC-overlay assays, SPR was used to observe the binding kinetics of phage antibodies. A sensorgram showing the binding of M3-7 phage to Man3-DPPE (the sensorgram was obtained by subtracting the binding of phage antibody to Man3-DPPE by that of DPPC alone) confirmed the Man3-specific binding property of the examined phage antibody (Fig. 4; sensorgram A). This result was further supported by the fact that (i) the binding of phage antibody was competitively inhibited by mannose (sensorgram C) or α-methyl-D-mannoside, and (ii) a control phage antibody which displayed human IGF-1 receptor specific scFv did not bind to Man3-DPPE (sensorgram B).

### (5) Expression and Characterization of Man3-Specific scFvs as Soluble Proteins

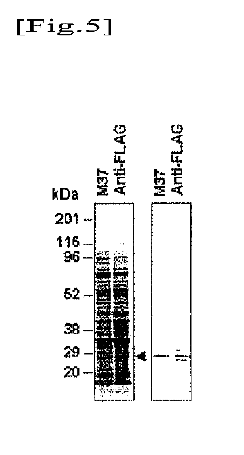

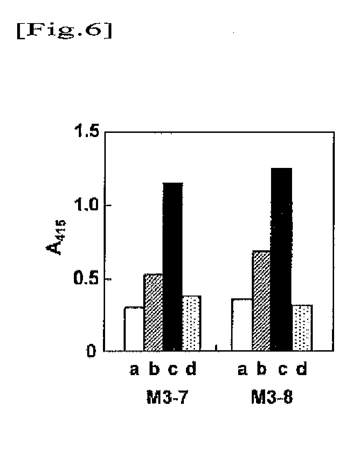

scFv proteins were expressed as soluble proteins for further analysis with regard to their carbohydrate specificity. The content of scFv proteins was compared among supernatants, periplasmic fractions, and whole cell extracts by SDS-PAGE and subsequent immunoblotting with anti-E-tag antibody. As a result, it was revealed that whole cell extract fractions contained the most scFv proteins expressed. The whole cell fractions were thus used for ELISA and SPR analyses (Fig. 5). Although the fractions used were unpurified (Fig. 5 Left), they contained E-tag reactive scFv proteins (Fig. 5 Right). The amounts of scFv proteins expressed by *E. coli* cells that had been infected with phages under the same condition to that of this study can also be considered to be within a similar range (Fig. 5 Right). The binding abilities of scFv proteins M3-7 and M3-8 to mannose residues were confirmed by ELISA with use of synthetic glycoproteins (Fig. 6).

### (6) SPR Analysis of Man3-Specific scFvs as Soluble Proteins

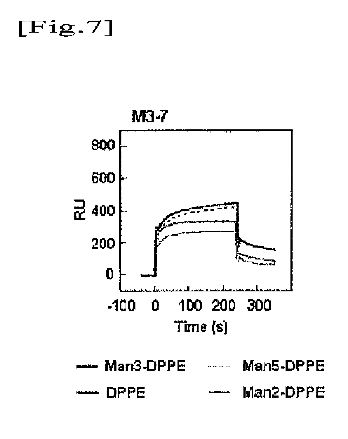

scFv protein samples (Fig. 5) were used to analyze the binding kinetics of Man3-specific scFv proteins by SPR using immobilized artificial glycolipids (Fig. 7). As a result, the scFv protein derived from the M3-7 phage clone showed similar relative kinetic of binding to Man3- DPPE and Man5-DPPE, which were higher than the binding kinetic to DPPE. On the other hand, the binding kinetic to Man2-DPPE was lower than the binding kinetic to DPPE (Fig. 7).

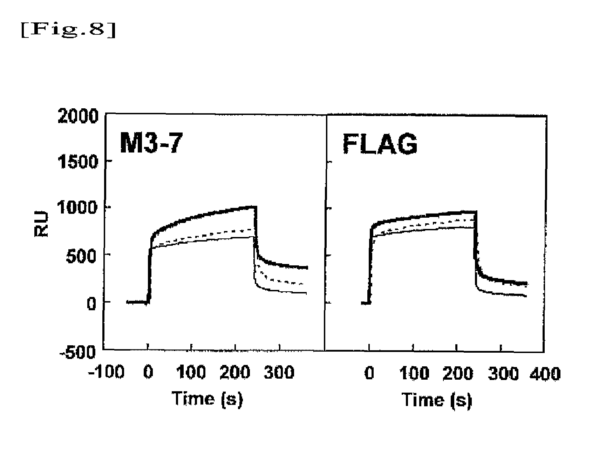

SPR Analyses of biotinylated carbohydrates were also carried out. In view of the binding kinetics of scFv protein samples to Man3-sp-biotin, Lewis b-sp-biotin, and glucose-sp-biotin, the scFv protein M3-7 bound to Man3 at much higher affinity than to Lewis b which is structurally irrelevant (the affinity of the scFv protein M3-7 to Lewis b was very low but significant) (Fig. 8). On the other hand, these scFv proteins did not appear to bind to glucose used as a control. The sensorgram of anti-FLAG scFv protein should be regarded as a control.

The sensorgrams of the bindings of scFv proteins to Man3-DPPE (Fig. 7) and Man3-biotin (Fig. 8) were respectively subtracted by the sensorgrams on DPPE (Fig. 9A) and glucose-biotin (Fig. 9B). From these two types of results, the specific binding to Man3 was compared (Fig. 9). These data show several facts regarding relative affinities between scFv proteins examined. For example, the scFv protein obtained from M3-7 has an affinity to Man3 structure.

### Industrial Applicability

The present invention provides antibodies which recognize non-reducing mannose residues, and nucleic acids encoding these antibodies. The antibodies of the present invention are able to bind, with specificity and high affinity, to various carbohydrates including carbohydrate antigens, individual recognition of which is not feasible. Further, gene manipulation of single chain antibodies of the present invention enables production of modified single chain antibodies, and mass production of single chain antibodies with ease. In addition, gene manipulation of the antibodies obtained by the present invention enables carbohydrate-specific delivery of proteins such as toxins and enzymes.

### Brief Description of the Drawings

Fig. 1 shows synthesis of artificial glycolipids (A) and Man3-DPPE (B). Artificial glycolipids were synthesized from oligosaccharides, such as Man1, Man2, Man3, Man5, GN2, LNT, FG, and GN2Man3 (whose structures are shown in Fig. 2C), and dipalmitoylphosphatidylethanolamine (DPPE) by reductive amination.
Fig. 2 shows carbohydrate specificity of phage antibodies screened by panning and ELISA with Man3-DPPE. (A) After various artificial glycolipids were dot-blotted on TLC plates, each plate was overlaid with anti-Man3 phage antibodies, M3-7, or M3-8, followed by detection of phages. (B) shows the artificial glycolipids used in this study. Man1, mannose; Man2, mannobiose (Manα1-6Man); Man3, mannotriose [Manα1-6(Manα1-3)Man]; Man5, mannopentaose[Manα1-6(Manα1-3)Manα1-6(Manα1-3)Man]; GN2Man3, GlcNAcβ-2Manα1-6(GlcNAcβ1-2Manα1-3)Man; GN2, di-N-acetylchitobiose (GlcNAcβ1-4GlcNAc); FG, Fucα1-2Gal; LNT, lacto-N-tetraose (Galβ1-3G1cNAcβ1-3Galβ1-4Glc)
Fig. 3 shows binding of phage antibodies to natural glycoproteins. Reactivity of M3-7 phage antibody to natural glycoproteins (fetuin, asialofetuin, RNase A, and RNase B) was examined by Western blotting. The protein bands were stained with Coomassie Brilliant Blue. Western blotting with M3-7 phage antibody and HRP-labeled anti-M13 phage antibody was carried out to identify the band where the phage antibodies was bound.
Fig. 4 shows SPR analysis of Man3-specific scFv-displaying phage antibodies. M3-7 phage antibody (A) was subjected to SPR analysis with Man3-DPPE. Anti-IGF-1 receptor scFv-phage was used as a negative control (B). A competitive inhibition test was performed with M3-7 phage antibody in the presence of 100 mM mannose (C). The sensorgrams shown are specific binding to Man3, which were obtained by subtracting the sensorgrams on DPPC from those obtained on specific Man3-DPPE.
Fig. 5 shows expression of Man3-specific scFv as a soluble protein. Left: SDS-PAGE of scFv protein (M3-7) expressed in *E. coli* TOP10F' as well as anti-FLAG scFv protein as a control was carried out. The gel was stained with Coomassie Brilliant Blue. Right: Western blotting of scFv proteins with an anti-E-tag antibody was performed.
Fig. 6 shows binding of scFv proteins to Man3-BSA (examined by ELISA). scFv proteins (M3-7 and M3-8) were analyzed by ELISA. Reactivities of respective scFv proteins to BSA (a), Man1-BSA (b), Man3-BSA (c), and scFv protein-free BSA (d) were determined.
Fig. 7 shows SPR analysis of Man3-specific scFv proteins on chip immobilized with Man2-DPPE, Man3-DPPE, or Man5-DPPE.
Fig. 8 shows SPR analysis of Man3-specific scFv proteins on chip immobilized with Man3-sp-biotin, Lewis b-sp-biotin, or glucose-sp-biotin. The thick line indicates Man3-sp-biotin; the dotted line indicates Lewis b-sp-biotin; and the thin line indicates glucose-sp-biotin.

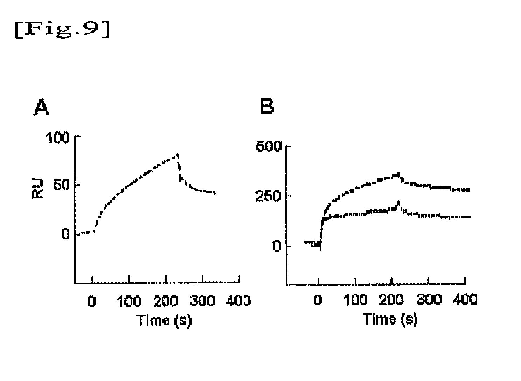

Fig. 9 shows comparison of the bindings of scFv proteins to Man3 moiety. The comparison was made between two types of sensorgrams of scFv proteins with respect to Man3-DPPE (Fig. 7) and Man3-biotin (Fig. 8). Specific bindings were obtained by subtracting the sensorgrams on DPPE (Fig. 9A) or glucose-biotin (Fig. 9B) from the respective sensorgrams.

## Claims

1. An antibody of any of the following (1) to (14):
(1) an antibody recognizing a non-reducing mannose residue, comprising
the amino acid sequence of SEQ ID NO: 2 or an amino acid sequence, wherein one or a few amino acids have been deleted, substituted, or added in the amino acid sequence of SEQ ID NO: 2, as an amino acid sequence of heavy chain variable region, and the amino acid sequence of SEQ ID NO: 4 or an amino acid sequence, wherein one or a few amino acids have been deleted, substituted, or added in the amino acid sequence of SEQ ID NO: 4, as an amino acid sequence of light chain variable region;
(2) an antibody recognizing a non-reducing mannose residue, comprising
the amino acid sequence of SEQ ID NO: 6 or an amino acid sequence, wherein one or a few amino acids have been deleted, substituted, or added in the amino acid sequence of SEQ ID NO: 6, as an amino acid sequence of heavy chain variable region, and the amino acid sequence of SEQ ID NO: 8 or an amino acid sequence, wherein one or a few amino acids have been deleted, substituted, or added in the amino acid sequence of SEQ ID NO: 8, as an amino acid sequence of light chain variable region;
(3) an antibody recognizing a non-reducing mannose residue, comprising
the amino acid sequence of SEQ ID NO: 10 or an amino acid sequence, wherein one or a few amino acids have been deleted, substituted, or added in the amino acid sequence of SEQ ID NO: 10, as an amino acid sequence of heavy chain variable region, and the amino acid sequence of SEQ ID NO: 12 or an amino acid sequence, wherein one or a few amino acids have been deleted, substituted, or added in the amino acid sequence of SEQ ID NO: 12, as an amino acid sequence of light chain variable region;
(4) an antibody recognizing a non-reducing mannose residue, comprising
the amino acid sequence of SEQ ID NO: 14 or an amino acid sequence, wherein one or a few amino acids have been deleted, substituted, or added in the amino acid sequence of SEQ ID NO: 14, as an amino acid sequence of heavy chain variable region, and the amino acid sequence of SEQ ID NO: 16 or an amino acid sequence, wherein one or a few amino acids have been deleted, substituted, or added in the amino acid sequence of SEQ ID NO: 16, as an amino acid sequence of light chain variable region;
(5) an antibody recognizing a non-reducing mannose residue, comprising
the amino acid sequence of SEQ ID NO: 18 or an amino acid sequence, wherein one or a few amino acids have been deleted, substituted, or added in the amino acid sequence of SEQ ID NO: 18, as an amino acid sequence of heavy chain variable region, and the amino acid sequence of SEQ ID NO: 20 or an amino acid sequence, wherein one or a few amino acids have been deleted, substituted, or added in the amino acid sequence of SEQ ID NO: 20, as an amino acid sequence of light chain variable region;
(6) an antibody recognizing a non-reducing mannose residue, comprising
the amino acid sequence of SEQ ID NO: 22 or an amino acid sequence, wherein one or a few amino acids have been deleted, substituted, or added in the amino acid sequence of SEQ ID NO: 22, as an amino acid sequence of heavy chain variable region, and the amino acid sequence of SEQ ID NO: 24 or an amino acid sequence, wherein one or a few amino acids have been deleted, substituted, or added in the amino acid sequence of SEQ ID NO: 24, as an amino acid sequence of light chain variable region;
(7) an antibody recognizing a non-reducing mannose residue, comprising
the amino acid sequence of SEQ ID NO: 26 or an amino acid sequence, wherein one or a few amino acids have been deleted, substituted, or added in the amino acid sequence of SEQ ID NO: 26, as an amino acid sequence of heavy chain variable region, and the amino acid sequence of SEQ ID NO: 28 or an amino acid sequence, wherein one or a few amino acids have been deleted, substituted, or added in the amino acid sequence of SEQ ID NO: 28, as an amino acid sequence of light chain variable region;
(8) an antibody recognizing a non-reducing mannose residue, comprising
the amino acid sequence of SEQ ID NO: 30 or an amino acid sequence, wherein one or a few amino acids have been deleted, substituted, or added in the amino acid sequence of SEQ ID NO: 30, as an amino acid sequence of heavy chain variable region, and the amino acid sequence of SEQ ID NO: 32 or an amino acid sequence, wherein one or a few amino acids have been deleted, substituted, or added in the amino acid sequence of SEQ ID NO: 32, as an amino acid sequence of light chain variable region;
(9) an antibody recognizing a non-reducing mannose residue, comprising
the amino acid sequence of SEQ ID NO: 34 or an amino acid sequence, wherein one or a few amino acids have been deleted, substituted, or added in the amino acid sequence of SEQ ID NO: 34, as an amino acid sequence of heavy chain variable region, and the amino acid sequence of SEQ ID NO: 36 or an amino acid sequence, wherein one or a few amino acids have been deleted, substituted, or added in the amino acid sequence of SEQ ID NO: 36, as an amino acid sequence of light chain variable region;
(10) an antibody recognizing a non-reducing mannose residue, comprising
the amino acid sequence of SEQ ID NO: 38 or an amino acid sequence, wherein one or a few amino acids have been deleted, substituted, or added in the amino acid sequence of SEQ ID NO: 38, as an amino acid sequence of heavy chain variable region, and the amino acid sequence of SEQ ID NO: 40 or an amino acid sequence, wherein one or a few amino acids have been deleted, substituted, or added in the amino acid sequence of SEQ ID: 40, as an amino acid sequence of light chain variable region;
(11) an antibody recognizing a non-reducing mannose residue, comprising
the amino acid sequence of SEQ ID NO: 42 or an amino acid sequence, wherein one or a few amino acids have been deleted, substituted, or added in the amino acid sequence of SEQ ID NO: 42, as an amino acid sequence of heavy chain variable region, and the amino acid sequence of SEQ ID NO: 44 or an amino acid sequence, wherein one or a few amino acids have been deleted, substituted, or added in the amino acid sequence of SEQ ID: 44, as an amino acid sequence of light chain variable region; and
(12) an antibody recognizing a non-reducing mannose residue, comprising
the amino acid sequence of SEQ ID NO: 46 or an amino acid sequence, wherein one or a few amino acids have been deleted, substituted, or added in the amino acid sequence of SEQ ID NO: 46, as an amino acid sequence of heavy chain variable region, and the amino acid sequence of SEQ ID NO: 48 or an amino acid sequence, wherein one or a few amino acids have been deleted, substituted, or added in the amino acid sequence of SEQ ID NO: 48, as an amino acid sequence of light chain variable region.

2. A nucleic acid encoding the antibody of claim 1.

3. A nucleic acid of any of the following:
(1) a nucleic acid comprising the nucleotide sequence of SEQ ID NO: 1 and the nucleotide sequence of SEQ ID NO: 3;
(2) a nucleic acid comprising the nucleotide sequence of SEQ ID NO: 5 and the nucleotide sequence of SEQ ID NO: 7;
(3) a nucleic acid comprising the nucleotide sequence of SEQ ID NO: 9 and the nucleotide sequence of SEQ ID NO: 11;
(4) a nucleic acid comprising the nucleotide sequence of SEQ ID NO: 13 and the nucleotide sequence of SEQ ID NO: 15;
(5) a nucleic acid comprising the nucleotide sequence of SEQ ID NO: 17 and the nucleotide sequence of SEQ ID NO: 19;
(6) a nucleic acid comprising the nucleotide sequence of SEQ ID NO: 21 and the nucleotide sequence of SEQ ID NO: 23;
(7) a nucleic acid comprising the nucleotide sequence of SEQ ID NO: 25 and the nucleotide sequence of SEQ ID NO: 27;
(8) a nucleic acid comprising the nucleotide sequence of SEQ ID NO: 29 and the nucleotide sequence of SEQ ID NO: 31;
(9) a nucleic acid comprising the nucleotide sequence of SEQ ID NO: 33 and the nucleotide sequence of SEQ ID NO: 35;
(10) a nucleic acid comprising the nucleotide sequence of SEQ ID NO: 37 and the nucleotide sequence of SEQ ID NO: 39;
(11) a nucleic acid comprising the nucleotide sequence of SEQ ID NO: 41 and the nucleotide sequence of SEQ ID NO: 43; and
(12) a nucleic acid comprising the nucleotide sequence of SEQ ID NO: 45 and the nucleotide sequence of SEQ ID NO: 47.

4. A process for producing an antibody wherein the nucleic acid of claim 3 is used.

5. A recombinant antibody recognizing a nonreducing mannose residue which can be obtained by the process of claim 4.

6. The recombinant antibody of claim 5 wherein the antibody is a single chain antibody.
